# EUROPEAN PATENT SPECIFICATION

(11) **EP 4 279 503 B1**
(45) Date of publication and mention of the grant of the patent: **13.08.2025**
(21) Application number: 22739501.9
(22) Date of filing: 14.01.2022
(51) Int. Cl.: G01N 33/569, C07K 16/12, C12N 15/13, C12Q 1/04, G01N 33/543

(54) **METHOD AND KIT FOR DETECTING PRESENCE AND/OR AMOUNT OF BACTERIA OF ENTEROBACTERIACEAE IN FOOD/DRINK SAMPLE, ENVIRONMENTAL SAMPLE, OR BIOLOGICAL SAMPLE**
VERFAHREN UND KIT ZUM NACHWEIS DER ANWESENHEIT UND/ODER DER MENGE VON BAKTERIEN AUS ENTEROBACTERIACEAE IN LEBENSMITTEL-/GETRÄNKEPROBE, UMWELTPROBE ODER BIOLOGISCHER PROBE
PROCÉDÉ ET KIT POUR DÉTECTER LA PRÉSENCE ET/OU LA QUANTITÉ DE BACTÉRIES D'ENTEROBACTERIACEAE DANS UN ÉCHANTILLON D'ALIMENT/BOISSON, UN ÉCHANTILLON ENVIRONNEMENTAL OU UN ÉCHANTILLON BIOLOGIQUE

(30) Priority: 15.01.2021 JP 2021004860; 15.01.2021 JP 2021004806
(43) Date of publication of application: 22.11.2023
(73) Proprietor: Asahi Kasei Kabushiki Kaisha, Tokyo 1000006 (JP)
(72) Inventor: TAKAHASHI, Katsuyoshi, Tokyo 100-0006 (JP); ODA, Kotaro, Tokyo 100-0006 (JP); SUGATA, Mika, Tokyo 100-0006 (JP)
(74) Representative: Forstmeyer, Dietmar
(86) International application number: PCT/JP2022/001206
(87) International publication number: WO 2022/154096

(56) References cited:
- EP-A1- 0 969 018
- EP-A1- 1 104 772
- WO-A2-2012/159023
- CN-A- 104 297 474
- JP-A- 2000 088 854
- JP-A- 2008 148 629
- JP-A- 2008 304 275
- JP-A- 2010 510 526
- JP-A- 2011 224 020
- JP-A- 2013 164 414
- JP-A- 2013 169 190
- JP-A- 2018 063 182
- JP-A- H06 324 039
- JP-B2- 2 594 622
- KR-A- 20200 070 792
- US-A1- 2009 269 789
- US-A1- 2020 041 510
- TATSUYA TOMINAGA: "Rapid detection of coliform bacteria using a lateral flow test strip assay", JOURNAL OF MICROBIOLOGICAL METHODS, vol. 160, 1 May 2019 (2019-05-01), NL, pages 29 - 35, XP093158089, ISSN: 0167-7012, DOI: 10.1016/j.mimet.2019.03.013

## Description

### TECHNICAL FIELD

The present invention relates a method and a kit for detecting the presence and/or amount of Enterobacteriaceae bacteria in a food or beverage sample, environmental sample, or biological sample.

### BACKGROUND ART

In the field of testing various types of samples, such as food or beverage samples, environmental samples, and biological samples, there is a particular demand to detect the presence or amount of bacteria belonging to the Enterobacteriaceae family (hereinafter referred to as "Enterobacteriaceae bacteria"). Conventional methods for determining the degree of contamination of food or beverage samples or environmental samples by Enterobacteriaceae bacteria include the culture method (e.g., Patent Literature 1: JP2003-116594 A), in which a sample is cultured to check bacterial growth, and the ATP method (e.g., Patent Literature 2: JP-H11-239493 A; and Patent Literature 3: JP2009-136205 A), in which intracellular ATP (adenosine triphosphate) of bacteria in a sample is detected.

Tatsuya Tominaga: "Rapid detection of coliform bacteria using a lateral flow test strip assay", JOURNAL OF MICROBIOLOGICAL METHODS, vol. 160, 1 May 2019 (2019-05-01), pages 29-35, relates to rapid detection of coliform bacteria using a lateral flow test strip assay.

However, the conventional culture method requires culture facilities and takes several days to a week before detection, which presents challenges in terms of space, labor, and time. On the other hand, the conventional ATP method has poor selectivity for Enterobacteriaceae bacteria, because ATP is present not only in Enterobacteriaceae bacteria but also in other bacterial cells. It also has difficulty in identifying Enterobacteriaceae bacteria from eukaryotic cells derived from foods and beverages, environment, and living organisms, because ATP is also present in eukaryotic cells (e.g., animal cells and plant cells).

There are actual situations in which the presence and/or amount of Enterobacteriaceae bacteria in a food or beverage sample, environmental sample, or biological sample should be detected, such as in bacterial tests for food or beverage and environmental sanitation, where it is necessary to detect bacteria of plural genera comprehensively and rapidly, rather than detecting various bacteria individually. In particular, since many Enterobacteriaceae bacteria cause food poisoning, etc., there are cases where unique control standards are established for inspections such as food and beverage hygiene inspections, environmental hygiene inspections, and food worker inspections. Accordingly, there is a high demand for comprehensive and rapid detection of Enterobacteriaceae bacteria. Therefore, if a method is provided that can collectively detect plural genera of Enterobacteriaceae bacteria present in food or beverage, environment, and living organisms, it will be of high technical significance and utility.

### LIST OF CITATIONS

### Patent Literature

[Patent Literature 1] JP2003-116594 A
[Patent Literature 2] JP-H11-239493 A
[Patent Literature 3] JP2009-136205 A

### SUMMARY OF INVENTION

### PROBLEM TO BE SOLVED BY THE INVENTION

An objective of an embodiment of the present invention is to provide a method and a kit capable of detecting the presence and/or amount of Enterobacteriaceae bacteria in food or beverage samples, environmental samples, and biological samples easily and efficiently in a short period of time.

### MEANS TO SOLVE THE PROBLEM

As a result of intensive study, the present inventors have found that simultaneous detection of the presence and/or amount of different Enterobacteriaceae bacteria of plural genera in a sample based on antigen-antibody reaction makes it possible to detect the presence and/or amount of Enterobacteriaceae bacteria in a sample easily and efficiently in a short period of time. The present inventors have also found that by using a generic antibody that causes antigen-antibody reactions widely with a broad spectrum of Enterobacteriaceae bacteria in combination with one or more specific antibodies that cause antigen-antibody reactions specifically with particular Enterobacteriaceae bacteria, it is possible to distinguish plural species of Enterobacteriaceae bacteria from other components in a sample and detect the presence and/or amount of the Enterobacteriaceae bacteria quickly and conveniently. The present inventors have then actually produced antibodies that cause antigen-antibody reactions with plural genera of Enterobacteriaceae bacteria and have demonstrated that these antibodies can be used to detect the presence and/or amount of the Enterobacteriaceae bacteria in a sample easily and efficiently in a short period of time, thereby arriving at the present invention.

The scope of protection is defined by the appended claims.

In detail, the invention pertains to a method for detecting the presence and/or amount of Enterobacteriaceae bacteria in a sample selected from food or beverage samples, environmental samples, and biological samples, the method comprising the step of simultaneously detecting the presence and/or amount of Enterobacteriaceae bacteria of two or more different genera in the sample based on antigen-antibody reactions, wherein the step of detecting comprises: contacting the sample with an antibody that causes antigen-antibody reactions with components derived from the Enterobacteriaceae bacteria of two or more genera; and measuring the presence and/or intensity of the antigen-antibody reaction that occurs in the sample after contact, wherein the antibody is a monoclonal antibody having: as a heavy chain variable region sequence, the amino acid sequence having a homology of 80% or more to the amino acid sequence of SEQ ID NO: 1, and as a light chain variable region sequence, the amino acid sequence having a homology of 80% or more to the amino acid sequence of SEQ ID NO:2; or as a heavy chain variable region sequence, the amino acid sequence having a homology of 80% or more to the amino acid sequence of SEQ ID NO:3, and as a light chain variable region sequence, the amino acid sequence having a homology of 80% or more to the amino acid sequence of SEQ ID NO:4; or as a heavy chain variable region sequence, the amino acid sequence having a homology of 80% or more to the amino acid sequence of SEQ ID NO:5, and as a light chain variable region sequence, the amino acid sequence having a homology of 80% or more to the amino acid sequence of SEQ ID NO:6.

The invention further pertains to a method for determining the degree of contamination by Enterobacteriaceae bacteria in a sample selected from food or beverage samples, environmental samples, and biological samples, comprising the step of simultaneously detecting the presence and/or amount of Enterobacteriaceae bacteria of two or more genera in the sample based on antigen-antibody reactions by the method according to any one of claims 1 to 10. The invention also pertains to a kit for detecting the presence and/or amount of Enterobacteriaceae bacteria in a sample selected from food or beverage samples, environmental samples, and biological samples by the method according to any one of claims 1 to 5, comprising an antibody as recited in any one of claims 1 to 5, as well as to a kit for detecting the presence and/or amount of Enterobacteriaceae bacteria in a sample selected from food or beverage samples, environmental samples, and biological samples by the method according to any one of claims 6 to 10, comprising a capture antibody and a detection antibody as recited in any one of claims 6 to 10, wherein one of the capture antibody and the detection antibody is the generic antibody and the other is the specific antibody, optionally the kit further comprises an insoluble membrane carrier for developing the sample and contacting the sample with the capture antibody, wherein the insoluble membrane carrier has a detection line formed thereon and the capture antibody is immobilized on the detection line, whereby two or more species of bacteria in the sample are detected on the single detection line.

Specific embodiments are mentioned in the dependent claims

### EFFECT OF THE INVENTION

The method according to the present invention makes it possible to detect the presence and/or amount of Enterobacteriaceae bacteria in food or beverage samples, environmental samples, and biological samples easily and efficiently in a short period of time.

### BRIEF EXPLANATION OF FIGURES

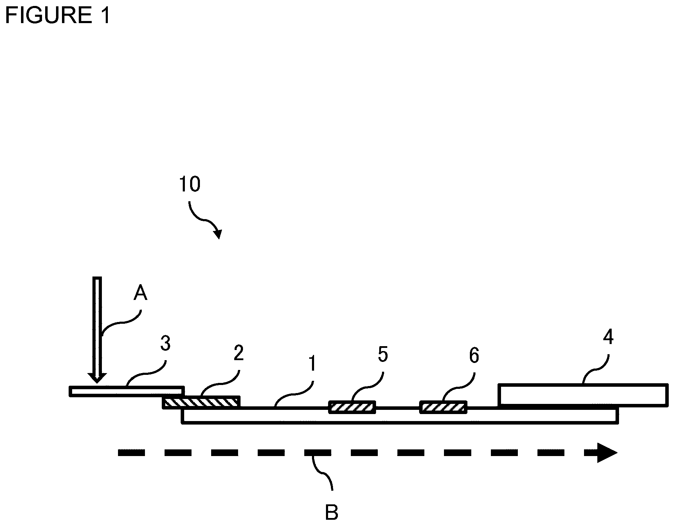

[Figure 1] Figure 1 is a cross-sectional view of a schematic diagram of a strip-shaped detection mechanism, an example of a detection mechanism of a lateral flow type immunochromatographic detection system.

### DESCRIPTION OF EMBODIMENTS

The present invention will be described in detail by referring to the specific embodiments below.

In the amino acid sequences described herein, each amino acid shall be represented by a single-letter code, unless otherwise specified.

One embodiment of the present invention relates to a method for detecting the presence and/or amount of Enterobacteriaceae bacteria in a food or beverage sample, environmental sample, or biological sample (hereinafter also referred to as "the method of the present invention"). The method of the present invention includes simultaneously detecting the presence and/or amount of Enterobacteriaceae bacteria of plural genera in the sample based on antigen-antibody reactions. According to one embodiment, such detection based on antigen-antibody reactions may be carried out by, e.g., contacting the sample with an antibody that causes antigen-antibody reactions with components derived from plural genera of Enterobacteriaceae bacteria in the sample (hereinafter also referred to as "the antibody of the present invention"), and measuring the presence and/or intensity of the antigen-antibody reactions that occur in the sample after contact. The method of the present invention makes it possible to determine the degree of contamination of, e.g., a food or beverage sample, environmental sample, or biological sample by Enterobacteriaceae bacteria easily and efficiently in a short period of time.

A kit comprising the antibody of the present invention for carrying out the method of the present invention (the kit of the present invention) constitutes another subject of the present invention.

In the following description, the method of the present invention will be explained first, followed by a description of the antibody used in the method of the present invention, then by a description of a not claimed method (2), and lastly by a description of the kit for use in the method of the present invention.

### [1. Method for Detecting the Presence and/or Amount of Enterobacteriaceae Bacteria in Sample (1)]

The method of the present invention is a method for detecting the presence and/or amount of Enterobacteriaceae bacteria in a sample selected from food or beverage samples, environmental samples, and biological samples. The method of the present invention includes simultaneously detecting the presence and/or amount of Enterobacteriaceae bacteria of plural genera in the sample based on antigen-antibody reactions. The method of the present invention may also be used for, e.g., determining the degree of contamination of the sample by Enterobacteriaceae bacteria.

Examples of samples include food or beverage samples, environmental samples, and biological samples (hereinafter, food or beverage samples and environmental samples may be referred to collectively as "food, beverage, or environmental samples," and food or beverage samples, environmental samples, and biological samples may be referred to collectively as "food, beverage, environmental, or biological samples"). The types of food or beverage samples are not particularly limited, but examples include samples obtained from food ingredients such as meat, fish, vegetables, processed foods and beverages, and seasonings, and beverages such as water, tea, coffee, juice, and alcoholic beverages. The types of environmental samples are also not particularly limited, but examples include samples obtained by wiping surfaces such as fingers, work clothes, work shoes, nail brushes, cutting boards, knives, handles, conveyor belts, packaging materials, work desks, beds, faucets, showers, and medical equipment in environments such as food and beverage manufacturing facilities, food and beverage serving sites, medical devices, medical equipment, and medical care sites with a collection tool (swab) such as clean cotton or clean cloth impregnated with a liquid medium (e.g., water, physiological isotonic solution, ethanol, etc.), as well as liquid samples such as tap water, well water, and river or hot spring water. The types of biological samples are also not particularly limited, but examples include samples derived from human or non-human animals, such as whole blood, serum, plasma, urine, stool, hands, saliva, sputum, sweat, nasal discharge, throat swab, nasal aspirate, and lung lavage fluid. Such food, beverage, environmental, or biological samples can be contaminated by a wide variety of Enterobacteriaceae bacteria.

As mentioned above, the culture method and the ATP method have conventionally been used to detect contamination of such food, beverage, environmental, or biological samples by Enterobacteriaceae bacteria. However, the culture method requires culture facilities and takes several days to a week before detection. The ATP method has difficulty in identifying Enterobacteriaceae bacteria from other bacteria and also from eukaryotic cells derived from foods and beverages, environment, and living organisms, because ATP is present not only in Enterobacteriaceae bacteria but also in eukaryotic cells (e.g., animal cells and plant cells).

The method of the present invention includes simultaneously detecting the presence and/or amount of Enterobacteriaceae bacteria of plural genera in the sample based on antigen-antibody reactions. The term "antigen-antibody reaction" used herein refers to the specific binding of an antibody to its antigen. The use of antigen-antibody reactions makes it possible to detect detecting the presence and/or amount of Enterobacteriaceae bacteria in the sample immediately on site using simple equipment and operation. The phrase "simultaneous" detection of plural species or plural genera of bacteria herein refers to plural species or plural genera of bacteria at once, and should not necessarily be limited to simultaneous detection in time. According to the present invention, by appropriately selecting and using antibodies (antibodies of the invention) that specifically generate antigen-antibody reactions with components derived from plural genera of Enterobacteriaceae bacteria to be detected, it is possible to simultaneously detect the plurality of genera of Enterobacteriaceae bacteria to be detected, while reducing false positives caused by components other than Enterobacteriaceae bacteria, thereby improving detection sensitivity and detection accuracy. As a result, the degree of contamination of the sample by plural genera of Enterobacteriaceae can be determined easily and efficiently in a short period of time.

Examples of Enterobacteriaceae bacteria to be detected in the present invention include Enterobacteriaceae bacteria belonging to the genus Escherichia, the genus Klebsiella, the genus Citrobacter, the genus Enterobacter, the genus Proteus, the genus Salmonella, and the genus Serratia (hereinafter also referred to as "specific Enterobacteriaceae genera"), which are representative Enterobacteriaceae genera with particularly high demand for detection in food, beverage, environmental, or biological samples. According to the method of the present invention, it may be preferable to detect antigen-antibody reactions with Enterobacteriaceae bacteria of at least two or more of these specific Enterobacteriaceae genera (plural genera), and especially preferable to detect antigen-antibody reactions with Enterobacteriaceae bacteria of at least three, or at least four, or at least five, or at least six, or at least seven of these genera.

Examples of Enterobacteriaceae bacteria belonging to the genus Escherichia include Escherichia coli (E. coli, EC) and Escherichia albertii (E. albertii). When the method of the present invention is used for detecting antigen-antibody reactions with Enterobacteriaceae bacteria belonging to the genus Escherichia, it is sufficient to detect antigen-antibody reactions with any one or more of these Enterobacteriaceae bacteria, but it may be preferred to detect antigen-antibody reactions with at least Escherichia coli (E. coli).

Examples of Enterobacteriaceae bacteria belonging to the genus Klebsiella include Klebsiella pneumoniae (K. pneumoniae, KP), Klebsiella oxytoca (K. oxytoca), and Klebsiella aerogenes (K. aerogenes). When the method of the present invention is used for detecting antigen-antibody reactions with Enterobacteriaceae bacteria belonging to the genus Klebsiella, it is sufficient to detect antigen-antibody reactions with any one or more of these Enterobacteriaceae bacteria, but it may be preferred to detect antigen-antibody reactions with at least Klebsiella pneumoniae.

Examples of Enterobacteriaceae bacteria belonging to the genus Citrobacter include Citrobacter freundii (C. freundii, CF), Citrobacter amalonaticus (C. amalonaticus), and Citrobacter diversus (C. diversus). When the method of the present invention is used for detecting antigen-antibody reactions with Enterobacteriaceae bacteria belonging to the genus Citrobacter, it is sufficient to detect antigen-antibody reactions with any one or more of these Enterobacteriaceae bacteria, but it may be preferred to detect antigen-antibody reactions with at least Citrobacter freundii.

Examples of Enterobacteriaceae bacteria belonging to the genus Enterobacter include Enterobacter cloacae (E. cloacae, ECL), Enterobacter aerogenes (E. aerogenes), and Enterobacter sakazakii (E. sakazakii). When the method of the present invention is used for detecting antigen-antibody reactions with Enterobacteriaceae bacteria belonging to the genus Enterobacter, it is sufficient to detect antigen-antibody reactions with any one or more of these Enterobacteriaceae bacteria, but it may be preferred to detect antigen-antibody reactions with at least Enterobacter cloacae.

Examples of Enterobacteriaceae bacteria belonging to the genus Proteus include Proteus milabilis (P. milabilis, PM), Proteus morganii (P. morganii), Proteus vulgaris (P. vulgaris), and Proteus rettgeri (P. rettgeri). When the method of the present invention is used for detecting antigen-antibody reactions with Enterobacteriaceae bacteria belonging to the genus Proteus, it is sufficient to detect antigen-antibody reactions with any one or more of these Enterobacteriaceae bacteria, but it may be preferred to detect antigen-antibody reactions with at least Proteus milabilis.

Examples of Enterobacteriaceae bacteria belonging to the genus Salmonella include Salmonella enteritidis (S. enteritidis, SE), Salmonella infantis (S. infantis), and Salmonella typhimurium (S. typhimurium). When the method of the present invention is used for detecting antigen-antibody reactions with Enterobacteriaceae bacteria belonging to the genus Salmonella, it is sufficient to detect antigen-antibody reactions with any one or more of these Enterobacteriaceae bacteria, but it may be preferred to detect antigen-antibody reactions with at least Salmonella enteritidis.

Examples of Enterobacteriaceae bacteria belonging to the genus Serratia include Serratia liquefaciens (S. liquefaciens, SL), Serratia marcescens (S. marcescens), and Serratia fonticola (S. fonticola). When the method of the present invention is used for detecting antigen-antibody reactions with Enterobacteriaceae bacteria belonging to the genus Serratia, it is sufficient to detect antigen-antibody reactions with any one or more of these Enterobacteriaceae bacteria, but it may be preferred to detect antigen-antibody reactions with at least Serratia liquefaciens.

According to one embodiment, the plurality of genera of Enterobacteriaceae bacteria may preferably include both one or more Gram-negative bacteria and one or more Gram-positive bacteria. Because the cell membrane and cell wall structures are very different between these groups, it is difficult to detect both groups of bacteria simultaneously using conventional technology. On the other hand, the method of the present invention allows for simultaneous detection of Gram-negative Enterobacteriaceae bacteria and Gram-positive Enterobacteriaceae bacteria, provided that the antibodies used for detection are appropriately designed.

The method of the present invention may be implemented so as not only to detect antigen-antibody reactions with Enterobacteriaceae bacteria belonging to the specific Enterobacteriaceae genera explained above, but also to detect antigen-antibody reactions with Enterobacteriaceae bacteria belonging to other one or more Enterobacteriaceae genera. Examples of Enterobacteriaceae bacteria belonging to other Enterobacteriaceae genera than the specific Enterobacteriaceae genera include, although are not limited to, Enterobacteriaceae bacteria belonging to the genus Yersinia, the genus Erwinia, the genus Hafnia, the genus Morganella, the genus Obesumbacterium, the genus Providencia, the genus Shigella, the genus Aeromonas, and the genus Pectobacterium (hereinafter also referred to as "selective Enterobacteriaceae genera"). Among these selective Enterobacteriaceae genera, it may be preferable to detect Enterobacteriaceae bacteria belonging to at least 2 or more genera, more preferably at least 3 or more genera, or at least 4 or more genera, or at least 5 or more genera, or at least 6 or more genera, or at least 7 or more genera, especially at least 8 or more, most preferably all 9 genera, of these selective Enterobacteriaceae genera.

According to the method of the present invention, compared to the conventional methods such as the culture method and the ATP method, it is possible to detect the presence and/or amount of Enterobacteriaceae bacteria in food, beverage, environmental, or biological samples more easily and efficiently in a shorter period of time, by simultaneously detecting antigen-antibody reactions with these Enterobacteriaceae bacteria. The method of the present invention also makes it possible, for example, to determine the degree of contamination of food, beverage, environmental, or biological samples with Enterobacteriaceae bacteria much more quickly and easily.

### [2. Antibodies that Cause Antigen-Antibody Reactions with Components Derived from Multiple Genera of Enterobacteriaceae Bacteria in Sample]

There are no restrictions to the means to be employed in the method of the present invention for simultaneously detecting the presence and/or amount of Enterobacteriaceae bacteria of plural genera in the sample based on antigen-antibody reactions. According to one embodiment, such detection based on antigen-antibody reactions may preferably be carried out by contacting the sample with a claimed antibody that causes antigen-antibody reactions with components derived from Enterobacteriaceae bacteria (the antibody of the present invention) and measuring the presence and/or intensity of the antigen-antibody reactions that occur in the sample after contact. The antibody will be explained below.

The term "antibody" used herein refers to a protein that recognizes and binds to a specific antigen or substance, which may also be referred to as an immunoglobulin (Ig). Common antibodies typically have two light chains (light chains) and two heavy chains (heavy chains) that are interconnected by disulfide bonds. There are two classes of light chains, called λ and κ chains, and five classes of heavy chains, called γ, µ, α, δ, and ε chains. Depending on the class of their heavy chains, antibodies are classified into five isotypes: IgG, IgM, IgA, IgD and IgE, respectively.

Heavy chains each include a heavy chain constant (CH) region and a heavy chain variable (VH) region. Light chains each include a light chain constant (CL) region and a light chain variable (VL) region. The light chain constant (CL) region consists of a single domain. The heavy chain constant (CL) region consists of three domains, namely CH1, CH2, and CH3. The light chain variable (VL) region and the heavy chain variable (VH) region each consist of four highly conserved regions called framework regions (FRs; FR-1, FR-2, FR-3, and FR-4) and three hypervariable regions called complementarity-determining regions (CDRs; CDR-1, CDR-2, and CDR-3). The heavy chain constant (CH) region consists of three CDRs (CDR-H1, CDR-H2, and CDR-H3) and four FRs (FR-H1, FR-H2, FR-H3, and FR-H4), which are arranged from the amino terminus to the carboxy terminus in the order of FR-H1, CDR-H1, FR-H2, CDR-H2, FR-H3, CDR-H3, and FR-H4. The light chain constant (CL) region has three CDRs (CDR-L1, CDR-L2, CDR-L3) and four FRs (FR-L1, FR-L2, FR L3, and FR-L4), which are arranged from the amino terminus to the carboxy terminus in the order of FR-L1, CDR-L1, FR-L2, CDR-L2, FR-L3, CDR-L3, and FR-L4. The variable regions of the heavy and light chains contain binding domains that interact with the antigen.

The antibody may be either a polyclonal antibody or a monoclonal antibody, but a monoclonal antibody be preferred. A polyclonal antibody is usually prepared from the serum of an animal immunized with an antigen and is a mixture of various antibody molecules with different structures. A monoclonal antibody, on the other hand, is an antibody composed of a single type of molecules containing a combination of light chain variable (VL) and heavy chain variable (VH) regions having determined amino acid sequences. Monoclonal antibodies can be produced from clones derived from antibody-producing cells, or they can be produced using genetic engineering technique, by obtaining nucleic acid molecules having gene sequences encoding amino acids of antibody proteins. It is also a well-known technique to those skilled in the art to improve the binding and specificity of antibodies using genetic information of their heavy chains and light chains or their variable regions and CDRs.

The antibody may be a fragment and/or derivative of an antibody. Fragments of antibodies include F(ab')₂, Fab, Fv, etc. Antibody derivatives include antibodies in which amino acid mutations have been artificially introduced into the constant region(s) of the light and/or heavy chains, antibodies in which the domain configuration of the constant region(s) of the light and/or heavy chains has been modified, antibodies with two or more Fc regions per molecule, glycosylated antibodies, bispecific antibodies, antibody conjugates in which an antibody or antibody fragment is bound to a protein other than the antibody, antibody enzymes, antibody conjugates in which an antibody or antibody fragment is bound to a protein other than an antibody, etc. Antibody conjugates, antibody enzymes, tandem scFv, bispecific tandem scFv, diabody, etc. Furthermore, when the aforementioned antibodies or their fragments or derivatives are derived from non-human animals, chimeric or humanized antibodies in which some or all of the sequences other than the CDRs thereof are replaced with the corresponding sequences of human antibodies are also included in the scope of the antibody of the present invention. When the simple term "antibodies" is used herein, it is intended to also encompass fragments and/or derivatives of antibodies, unless otherwise specified.

When the antibody causes antigen-antibody reactions with certain bacteria, it means that they bind specifically to some components of the bacteria as antigens. The components of Enterobacteriaceae bacteria that serve as antigens for the antibody are not limited. It may be a component contained in the cell walls or cell membranes that are exposed outside the bacterial cells, or it may be a component contained in the cytoplasm, cell organelles, or nucleus and not exposed outside the bacterial cells. When the antibody causes antigen-antibody reactions with components of Enterobacteriaceae bacteria that are not exposed outside the cell surface of the bacteria, the food, beverage, environmental, or biological sample may be subjected to a treatment for lysing the bacteria before being brought into contact with the antibody of the present invention. Such bacterial lysis treatment will be described later.

The antibody may preferably cause antigen-antibody reactions with Enterobacteriaceae bacteria belonging to at least 2 or more, or 3 or more, or 4 or more, or 5 or more, or 6 or more, or 7 or more genera selected from the group consisting of the genus Escherichia, the genus Klebsiella, the genus Citrobacter, the genus Enterobacter, the genus Proteus, the genus Salmonella, and the genus Serratia. The specific bacterial species belonging to these genera are described above.

According to one embodiment, the antibody may preferably cause antigen-antibody reactions with ribosome proteins, especially ribosome proteins L7/L12, of Enterobacteriaceae bacteria belonging to any of the specific Enterobacteriaceae genera mentioned above. The antibody may preferably cause antigen-antibody reactions also with ribosome proteins L7/L12 of Enterobacteriaceae bacteria belonging to any of the selective Enterobacteriaceae genera mentioned above. As used herein, the term "ribosome protein L7/L12," or simply "L7/L12," refers to a type of ribosomal protein essential for microbial protein synthesis and commonly possessed by various bacteria. For antibodies that cause antigen-antibody reactions against the bacterial ribosomal proteins L7/L12 and methods for producing the same, reference may be made to, e.g., to WO2000/006603A, the publication of an earlier patent application filed by the present inventors.

The components of Enterobacteriaceae bacteria that serve as antigens for the antibody of the present invention are not limited. They may be components contained in the cell walls or cell membranes that are exposed outside the bacterial cells, or they may be components that are contained in the cytoplasm, cell organelles, or nucleus and not exposed outside the bacterial cells. When the antibody causes antigen-antibody reactions with components of Enterobacteriaceae bacteria that are not exposed outside the cell surface of the bacteria, the food, beverage, environmental, or biological sample may be subjected to a treatment for lysing the bacteria before being brought into contact with the antibody of the present invention. Such bacterial lysis treatment will be described later.

The degree of antigen-antibody reactions between the antibodies and Enterobacteriaceae bacteria is not particularly limited, but it is sufficient that at least antigen-antibody reactions occur to the extent that it can be detected by any known detection method. Methods for detecting antigen-antibody reactions between antibodies and Enterobacteriaceae bacteria are not limited, but include various known immunological assays as described below.

The antibody may preferably not cause any cross-reactions with components derived from one or more bacteria other than Enterobacteriaceae bacteria (herein also referred to as "non-Enterobacteriaceae bacteria") that may be present in the sample. Examples of such non-Enterobacteriaceae bacteria include, although are not limited to, one or more bacteria selected from the genus Pseudomonas, the genus Staphylococcus, the genus Bacillus, and the genus Enterococcus. The antibody may preferably not cause any cross-reactions with components derived from at least one, or 2 or more, usually 3 or more, 4 or more, or 5 or more, or 6 or more, or 7 or more, or 8 or more, especially 9 or more, most preferably 10 or more, species selected from these non-Enterobacteriaceae bacteria.

The antibody may also preferably not cause any cross-reactions with one or more non-bacterial components that may be present in the sample. Examples of such non-bacterial components include, although are not limited to, various bioorganic compounds derived from viruses, plants, and/or animals that are not present in Enterobacteriaceae bacteria. Specific examples of such bioorganic compounds include proteins, sugars, glycoproteins, lipids, complex lipids, and nucleic acids. The antibody may preferably not cause any cross-reactions with components derived from at least one, or 2 or more, or 3 or more, or 4 or more, or 5 or more, or 6 or more, or 7 or more, or 8 or more, especially 9 or more, most preferably 10 or more, of these non-bacterial organic compounds.

Because antibodies are extremely antigen-specific, they are effective in specifically capturing certain antigens, but were considered unsuitable for, e.g., detecting plural different target substances. Moreover, since Enterobacteriaceae species that need to be detected in food or beverage, environmental, or biological tests are extremely diverse, it has conventionally been considered extremely difficult to simultaneously detect plural species of Enterobacteriaceae bacteria by antigen-antibody reactions. However, as will be described in the Examples below, the present inventors have succeeded in obtaining antibodies that cause antigen-antibody reactions with plural species of Enterobacteriaceae bacteria that can be the target for detection when testing food or beverage, environmental, or biological samples, and that can be used for simultaneous detection of these Enterobacteriaceae bacteria. This is an extremely surprising finding that goes against conventional technical knowledge.

Specifically, the antibody may have any of the amino acid sequences mentioned below as the amino acid sequences of each variable region of the heavy and light chains.

The heavy chain variable region (VH) may preferably have the amino acid sequence having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence selected from SEQ ID NO:1, SEQ ID NO:3, and SEQ ID NO:5. The VH sequence may more preferably be the amino acid sequence selected from SEQ ID NO:1, SEQ ID NO:3, and SEQ ID NO:5.

The light chain variable region (VL) may preferably have the amino acid sequence having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence selected from SEQ ID NO:2, SEQ ID NO:4, and SEQ ID NO:6. The VL sequence may more preferably be the amino acid sequence selected from SEQ ID NO:2, SEQ ID NO:4, and SEQ ID NO:6.

The following combinations may particularly be preferred.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:1 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:2.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:3 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:4.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:5 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:6.

As used herein, the term "homology" between two amino acid sequences refers to the ratio in which identical or similar amino acid residues appear in each corresponding position when these amino acid sequences are aligned, and the term "identity" between two amino acid sequences refers to the ratio of similar amino acid residues appearing in each corresponding position when these amino acid sequences are aligned. The "homology" and "identity" between two amino acid sequences can be determined using, for example, the BLAST (Basic Local Alignment Search Tool) program (Altschul et al., J. Mol. Biol., (1990), 215(3):403-10). (1990, 215(3):403-10).

Methods for identifying the sequence of each CDR from the respective variable sequences of the heavy and light chains of an antibody include, for example, the Kabat method (Kabat et al., The Journal of Immunology, 1991, Vol. 147, No. 5, pp. 1709-1719) and the Chothia method (Al Lazikani et al., Journal of Molecular Biology, 1997, Vol. 273, No. 4, pp. 927-948). These methods are common knowledge in this field, but it is possible to refer to, for example, the website of Dr. Andrew C.R. Martin's Group (http://www.bioinf.org.uk/abs/).

Similar amino acids include, for example, amino acids that are classified under the same group in the following classification based on the polarity, charge, and size of each amino acid (in any case, each amino acid type is indicated by a single-letter code).
*Aromatic amino acids: F, H, W, Y;
*Aliphatic amino acids: I, L, V;
*Hydrophobic amino acids: A, C, F, H, I, K, L, M, T, V, W, Y;
*Charged amino acids: D, E, H, K, R, etc.;
*Positively charged amino acids: H, K, R;
*Negatively charged amino acids: D, E;
*Polar amino acids: C, D, E, H, K, N, Q, R, S, T, W, Y;
*Small amino acids: A, C, D, G, N, P, S, T, V, etc.; and
*Micro amino acids: A, C, G, S.

Similar amino acids also include, for example, amino acids that are classified under the same group in the following classification based on the side chain of each amino acid (in any case, each amino acid type is indicated by a single-letter code).
*Amino acids having an aliphatic side chain: G, A, V, L, I;
*Amino acids having an aromatic side chain: F, Y, W;
*Amino acids having a sulfur-containing side chain: C, M;
*Amino acids having an aliphatic hydroxyl side chain: S, T;
*Amino acids having a basic side chain: K, R, H;
*Amino acids having an acidic side chain and their amide derivatives: D, E, N, Q.

The method of producing the antibody is not restricted. In the case of polyclonal antibodies, they can be prepared using components derived from Enterobacteriaceae bacteria to be detected. The components derived from Enterobacteriaceae that can be used include bacterial cells, lysate made by lysing bacterial cells, and a fraction of lysate obtained via electrophoresis. When an electrophoretic fraction of bacterial lysate is used, there are no restrictions on which fraction should be used, but it is preferable to select a fraction that corresponds to a molecular weight of approximately 10 to 20 kDa, for example. In addition, it is preferable to use ribosomal proteins contained in Enterobacteriaceae bacteria as components derived from Enterobacteriaceae bacteria, especially ribosomal proteins L7/L12. These Enterobacteriaceae-derived components are inoculated into an animal, optionally together with adjuvants as necessary, and the serum is collected to obtain antiserum containing antibodies (polyclonal antibodies) that causes antigen-antibody reactions with the plural species of Enterobacteriaceae bacteria mentioned above. Examples of animals to be inoculated include sheep, horses, goats, rabbits, mice, rats, etc., of which sheep and rabbits are especially preferred for polyclonal antibody production. The antibodies may be purified and fractionated from the obtained antiserum and screened for antigen-antibody reactivity with the desired Enterobacteriaceae bacteria, and for no cross-reactivity with other components of food or beverage, environment, or biological origin, using known methods to obtain the desired antibodies with superior specificity. Furthermore, monoclonal antibodies can be obtained by isolating antibody-producing cells that produce desired antibody molecules and fusing them with myeloma cells to produce hybridomas capable of autonomous growth. As a method that does not require sensitization of animals, a phage library expressing heavy-chain variable (VH) regions or light-chain variable (VL) regions of antibodies or parts thereof can be screened for phage clones expressing specific amino acid sequences that specifically bind to components derived from Enterobacteriaceae bacteria to be detected, and antibodies can be produced using information of the screened phage clones.

Once the desired antibodies are obtained by the above procedure, the structure of each antibody, specifically part or all of the amino acid sequences of the heavy chain constant (CH) region, heavy chain variable (VH) region, light chain constant (CL) region, and/or light chain variable (VL) region, can be analyzed using known amino acid sequence analysis methods. It is also known to those skilled in the art to modify the amino acid sequences of the desired antibodies thus obtained to improve their binding ability and specificity. Furthermore, it is also possible to design other antibodies likely to have similar antigen specificity by using all or part of the amino acid sequences of each desired antibody (especially the amino acid sequences of all or part of the heavy chain variable (VH) region and the light chain variable (VL) region, especially the amino acid sequence of each CDR) and, if necessary, by combining them with part of amino acid sequences of known antibodies (especially the amino acid sequences of each FR of the heavy chain variable (VH) and light chain variable (VL) regions, and optionally of the heavy chain variable (VH) and light chain variable (VL) regions).

Once the amino acid sequences of a desired antibody are determined, a nucleic acid molecule having base sequences encoding all or part of the amino acid sequences of the desired antibody can be produced by a known method, and the antibody can be produced by genetic engineering using such a nucleic acid molecule. Furthermore, it is also possible to create a vector or plasmid for expressing each component of the desired antibody from such a nucleic acid sequence, and introduce it into a host cell (e.g., a mammalian cell, insect cell, plant cell, yeast cell, or microbial cell) to produce the antibody. In addition, modifications can be made to the structures of the constant regions of the antibody or to its sugar chains in order to improve the efficiency of the obtained antibody or to avoid its side effects, using techniques well known to those skilled in the art.

Those explained above, i.e., methods for producing the antibody, nucleic acid molecules encoding the antibody, vectors or plasmids containing such nucleic acid molecules, cells containing such nucleic acid molecules, vectors or plasmids, hybridomas producing the antibodies etc., are also disclosed.

The techniques for making and modifying the antibodies described herein are all known to those skilled in the art, and may be carried out by referring to, e.g., Antibodies; A laboratory manual, E. Harlow et al. Likewise, the molecular biological techniques described herein (e.g., amino acid sequencing methods, methods for designing and producing nucleic acid molecules, methods for designing and producing vectors and plasmids, etc.) are also all known to those skilled in the art, and may be carried out by referring to, e.g., Molecular Cloning, A laboratory manual, Cold Spring Harbor Laboratory Press, Shambrook, NY. Harbor Laboratory Press, Shambrook, J. et al.

Thus, a preferred embodiment of the method of the present invention includes using an antibody that recognizes ribosomal proteins of each Enterobacteriaceae bacterium and causes antigen-antibody reactions (the antibody of the present invention), bringing them into contact with the sample, and detecting antigen-antibody reactions. In this embodiment, the detection sensitivity can be improved by exposing the ribosomal proteins of Enterobacteriaceae bacteria present in the sample to outside the bacterial cell membranes before bringing the sample into contact with the antibody of the present invention. Thus, in the preferred embodiment of the method of the present invention using the antibody, the sample may preferably be subjected to treatment for lysing Enterobacteriaceae bacteria before being brought into contact with the antibody of the present invention. Examples of the lysis treatment of Enterobacteriaceae bacteria include, although are not limited to, heating treatment, ultrasonication, and chemical treatment using a surfactant. Conditions for the lysis treatment may be determined as appropriate depending on the bacteria contained in the sample. In the preferred embodiment of the method of the present invention using the antibody, the antibody of the present invention may be brought into contact with the food, beverage, environmental, or biological sample by any arbitrary means.

In the preferred embodiment of the method of the present invention using the antibody, immunological assays for detecting antigen-antibody reactions are not limited. Examples of immunological assays are not limited and may be either methods using a single antibody or those using two or more antibodies.

Examples of immunological assays using a single antibody include, although are not limited to, various known immunological assays such as ELISA (enzyme-linked immunosorbent assay) using microtiter plates loaded with bacterial antigens to detect antigen-antibody reactions with antibodies; and biosensors that have antibodies (or antigens) loaded on the sensor surface to detect antigen-antibody reactions with antigens (or antibodies) electrically (e.g., AC impedance method, FET (field effect transistor) method) or optically (SPR (surface plasmon resonance) method). Any of these assays can be used in the method of the present invention.

Examples of immunological assay methods using two or more antibodies include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid phase carrier such as magnetic particles, etc. In the case of immunological assays that combine two or more antibodies, such as the sandwich assay, in which a detection antibody and a capture antibody are used, the antibody of the present invention may be used either as the capture antibody or as the detection antibody. Unless otherwise specified, the term "specific antibodies" as used herein refers to antibodies that cause antigen-antibody reactions with specific bacteria, including two or more Enterobacteriaceae species (bacteria to be detected) that are the final targets of detection, while the term "generic antibodies" as used herein refers to antibodies that cause antigen-antibody reactions with bacteria of five or more genera, including the aforementioned bacteria to be detected

### [3. Method for Detecting the Presence and/or Amount of Enterobacteriaceae Bacteria in Sample (2)]

It may be preferred to use, as a sandwich assay, a method of detecting the presence and/or amount of the bacteria in the sample by capturing the bacteria in the sample and labeling the bacteria in the sample based on antigen-antibody reactions between the sample, a capture antibody bound to a solid-phase carrier, and a detection antibody having a detection label, and detecting the bacteria to be detected in the sample based on the detection label. This method (hereinafter also referred to as "the method (2)")will be explained below.

In the method (2), the specific antibody may preferably be an antibody that causes antigen-antibody reactions with bacteria of at least 1 or more, or 2 or more, or 3 or more, or 4 or more, or 5 or more, or 6 or more, or 7 or more genera selected from the genus Escherichia, the genus Klebsiella, the genus Citrobacter, the genus Enterobacter, the genus Proteus, the genus Salmonella, and the genus Serratia.

In the method (2), the generic antibody may preferably be an antibody that causes antigen-antibody reactions with bacteria of at least 5 or more, or 6 or more, or 7 or more, or 8 or more, or 9 or more, or 10 or more, or 11 or more genera selected from the genus Escherichia, the genus Klebsiella, the genus Citrobacter, the genus Enterobacter, the genus Proteus, the genus Salmonella, the genus Serratia, the genus Pseudomonas, the genus Staphylococcus, the genus Bacillus, and the genus Enterococcus.

In the method (2), the structures of the specific antibody and the generic antibody are as described in the claims. These antibodies-have as the amino acid sequence of each variable region of the heavy and light chains, any of the following amino acid sequences.

The heavy chain variable region (VH) of the specific antibody may preferably have an amino acid sequence with a homology (preferably identity) of 80% or more, particularly 85% or more, more particularly 90% or more, especially 95% or more, or 96% or more, or 97% or more, or 98% or more, or 99% or more, most preferably 100%, with the amino acid sequence selected from SEQ ID NO:1, SEQ ID NO:3, and SEQ ID NO:5. The VH sequence may more preferably be the amino acid sequence selected from SEQ ID NO:1, SEQ ID NO:3, and SEQ ID NO:5.

The light chain variable region (VL) of the specific antibody may preferably have the amino acid sequence with a homology (preferably identity) of 80% or more, particularly 85% or more, more particularly 90% or more, especially 95% or more, or 96% or more, or 97% or more, or 98% or more, or 99% or more, most preferably 100%, with the amino acid sequence selected from SEQ ID NO:2, SEQ ID NO:4, and SEQ ID NO:6. The VL sequence may more preferably be the amino acid sequence selected from SEQ ID NO:2, SEQ ID NO:4, and SEQ ID NO:6.

The following combinations may particularly be preferred.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:1 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:2.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:3 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:4.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:5 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:6.

The heavy chain variable region (VH) of the generic antibody may preferably have an amino acid sequence with a homology (preferably identity) of 80% or more, particularly 85% or more, more particularly 90% or more, especially 95% or more, or 96% or more, or 97% or more, or 98% or more, or 99% or more, most preferably 100%, with an amino acid sequence selected from SEQ ID NO:7 and SEQ ID NO:9. The VH sequence may more preferably be an amino acid sequence selected from SEQ ID NO:7 and SEQ ID NO:9.

The light chain variable region (VL) of the generic antibody may preferably have an amino acid sequence with a homology (preferably identity) of 80% or more, particularly 85% or more, more particularly 90% or more, especially 95% or more, or 96% or more, or 97% or more, or 98% or more, or 99% or more, most preferably 100%, with an amino acid sequence selected from SEQ ID NO:8 and SEQ ID NO: 10. The VL sequence may more preferably be an amino acid sequence selected from SEQ ID NO:8 and SEQ ID NO: 10.

Preferred combinations of amino acid sequences of heavy chain variable (VH) and light chain variable (VL) regions of the generic antibody include, although are not limited to, possible combinations of a heavy chain variable region (VH) having an amino acid sequence with 80% or more homology (preferably identity) with any one amino acid sequence selected from SEQ ID NO:7 and SEQ ID NO:9 and a light chain variable region (VL) having an amino acid sequence with 80% or more homology (preferably identity) with any one amino acid sequence selected from SEQ ID NO:8 and SEQ ID NO:10. However, any of the following combinations may particularly be preferred.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:7 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:8.

Combinations of a heavy chain variable region (VH) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:9 and a light chain variable region (VL) having a homology (preferably identity) of 80% or more, particularly 85% or more, more particularly 90% or more, especially 95% or more, or 96% or more, or 97% or more, or 98% or more, or 99% or more, most preferably 100%, with the amino acid sequence of SEQ ID NO:10.

The method (2) is a method for detecting the presence and/or amount of bacteria in the sample by (I) capturing the bacteria in the sample and detecting the bacteria in the sample based on antigen-antibody reactions between the sample, a capture antibody bound to a solid-phase carrier, and a detection antibody having a detection label, and (II) detecting the Enterobacteriaceae bacteria to be detected in the sample based on the detection label. This method is further characterized in that one of the capture antibody and the detection antibody is at least one specific antibody, which causes antigen-antibody reactions with two or more bacteria to be detected, and the other is bacteria of five or more genera including the bacteria to be detected causes antigen-antibody reactions with, at least one generic antibody.

One example of the method (2) may be characterized in that Step (I) includes the steps of:
(Ia-1) contacting the sample with the detection antibody and detecting the bacteria in the sample based on antigen-antibody reactions between the detection antibody and bacteria, and
(Ia-2) contacting the capture antibody with the sample containing the bacteria labeled by the detection antibody and capturing the bacteria in the sample based on antigen-antibody reactions between the capture antibody and the bacteria-detection antibody complex.

Another example may be characterized in that Step (I) includes the steps of:
(Ib-1) contacting the sample with the capture antibody and capturing the bacteria in the sample based on antigen-antibody reactions between the capture antibody and bacteria, and
(Ib-2) contacting the detection antibody with the sample containing the bacteria captured by the capture antibody and detecting the bacteria in the sample based on antigen-antibody reactions between the detection antibody and the bacteria-capture antibody complex.

In either example, the capture antibody may be the generic antibody and the detection antibody may be the specific antibody. Alternatively, the detection antibody may be the generic antibody and the capture antibody may be the specific antibody. In either case, the antibody may be either the generic antibody or the specific antibody. According to one embodiment of the present invention, the antibody may preferably be, although is not limited to, used as the specific antibody. Specifically, when the antibody is used as the generic antibody, it is possible to prepare, as the generic antibody, an antibody that has relatively low specificity, i.e., that causes antigen-antibody reactions with a wide variety of Enterobacteriaceae bacteria (usually 5 or more, 6 or more, 7 or more, 8 or more, 9 or more, 10 or more, or 11 or more genera), including the Enterobacteriaceae bacteria that are the final target of detection, and to use it in combination with, as the specific antibody, an antibody with relatively high specificity, i.e., that causes antigen-antibody reactions only with the Enterobacteriaceae bacteria that are the final target of detection, and also does not antigen-antibody reactions with bacteria of other genera. On the other hand, when the antibody is used as the specific antibody, it is possible to prepare, as the specific antibody, an that has relatively high specificity, i.e., that causes antigen-antibody reactions only with the Enterobacteriaceae bacteria that are the final target of detection and does not cause antigen-antibody reactions with bacteria of other genera, and to use it in combination with, as the generic antibody, an antibody with relatively low specificity, i.e., that causes antigen-antibody reactions with a wide variety of Enterobacteriaceae bacteria (usually 5 or more, 6 or more, 7 or more, 8 or more, 9 or more, 10 or more, or 11 or more genera), including the Enterobacteriaceae bacteria that are the final target of detection.

In the method (2), either example may be chosen according to the type of immunoassay method actually used and the type of samples. Examples of immunoassay methods include, although are not limited to, various known immunological assays such as ELISA (enzyme-linked immunosorbent assay) using microtiter plates loaded with bacterial antigens to detect antigen-antibody reactions with antibodies; and biosensors that have antibodies (or antigens) loaded on the sensor surface to detect antigen-antibody reactions with antigens (or antibodies) electrically (e.g., AC impedance method, FET (field effect transistor) method) or optically (SPR (surface plasmon resonance) method).

The following explanation will be given based on an example carried out using immunochromatography as the immunoassay. Each feature may be modified as appropriate when other types of immunoassay methods are used.

In Step (Ia-1) above, i.e., the step of contacting the sample with the detection antibody and labeling the bacteria in the sample based on antigen-antibody reactions between the detection antibody and bacteria, the detection antibody with the label for detection is brought into contact with the sample, so that the bacteria in the sample are labeled based on antigen-antibody reactions between the detection antibody and the bacteria. The method for bringing the sample into contact with the detection antibody is not limited, but this may typically be carried out by introducing the sample prepared as an aqueous sample onto a member area impregnated with the detection antibody, and maintaining it for a certain period of time. Although this can be achieved by a variety of specific embodiments depending on, e.g., the mode of capture in Step (a), one example includes preparing a solid-phase carrier (porous membrane) on which the capture antibody is immobilized, preparing a conjugate pad to which the detection antibody is attached, placing the conjugate pad upstream of the solid-phase carrier, and introducing the sample prepared as an aqueous sample onto the conjugate pad and allowing it to pass through, thereby bringing the sample into contact with the detection antibody. As another example, when a flow channel is used as the solid-phase carrier, the sample prepared as an aqueous sample may be brought into contact with the detection antibody either upstream of the position on the flow channel where the capture antibody is immobilized or before the introduction of the solid-phase carrier.

In Step (Ia-2) above, i.e., the step of contacting the capture antibody with the sample containing the bacteria labeled by the detection antibody and capturing the bacteria in the sample based on antigen-antibody reactions between the capture antibody and the bacteria-detection antibody complex, the capture antibody is brought into contact with the sample, so that the bacteria in the sample is captured based on antigen-antibody reactions between the capture antibody and the bacteria. The method for bringing the sample into contact with the capture antibody is not limited, but this may typically be carried out by introducing the sample prepared as an aqueous sample onto an area where the capture antibody is present, and maintaining it for a certain period of time. Although this can be achieved by a variety of specific embodiments depending on, e.g., the type of solid-phase carrier of the capture antibody, one example includes using a porous membrane as the solid-phase carrier, and introducing the bacteria-detection antibody complex onto the porous membrane to which the capture antibody is immobilized and allowing it to permeate, such that the bacteria in the sample is captured by the capture antibody immobilized on the porous membrane. Another example includes immobilizing the capture antibody on an area of a flow channel used as the solid-phase carrier, and allowing the bacteria-detection antibody complex to pass through the flow channel to allow the capture antibody immobilized on the area of the flow channel to capture the bacteria in the sample.

In Step (II) above, i.e., the step detecting the bacteria to be detected in the sample based on the detection label, the bacteria to be detected captured by the capture antibody and labeled with the detection antibody is detected based on the detection label. The detection method is not restricted and may be selected depending on the type of detection label. For example, when a metallic colloid such as gold colloid is used as the detection label, the presence or amount of gold colloid bound to the target bacteria may be detected by any method such as visual inspection or using a camera.

According to the method (2), it is possible to distinguish the desired Enterobacteriaceae bacteria from other bacteria or other components in the sample and to detect them easily and efficiently, by appropriately combining a generic antibody, which causes antigen-antibody reactions widely with various species of bacteria, and a specific antibody, which causes antigen-antibody reactions specifically with a particular species of Enterobacteriaceae bacteria. Specifically, by using an appropriate combination of plural specific antibodies, it is possible to design a system for detecting various desired combinations of Enterobacteriaceae bacteria. Any immunoassay method can be used to perform method (2), as long as it employs capture and detection antibodies.

### [4. Kit for Detecting the Presence and/or Amount of Enterobacteriaceae Bacteria in the Sample]

As mentioned above, a kit for use in the method of the present invention containing the antibodies used in the claimed methods (the kit of the invention) is also included in the subject of the present invention.

The kit of the present invention includes, in addition to the antibody, one or more reagents and a device for detection or components thereof necessary to perform the method of the present invention using the antibody, and/or instructions describing the procedure for performing the method of the present invention. The type of such reagents and instructions, as well as other components included in the kit of the present invention, may be determined according to the specific immunological assay used to detect Enterobacteriaceae bacteria of plural genera.

When the kit of the present invention contains a device for detection or components thereof, the device assembled from the kit is a device equipped with components necessary for performing the method of the present invention using the antibody (hereinafter also referred to as "the device of the present invention"). The specific components of the device of the present invention may be determined according to the type of immunological assay as a specific embodiment of the method of the present invention. As explained above, examples of immunological assay methods using two or more antibodies include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid phase carrier such as magnetic particles, ELISA using a single antibody, and biosensor methods. Devices equipped with components necessary to perform such various immunological assays may be used as the device of the present invention.

Specific examples of devices capable of simultaneously and simply detecting the presence and/or amount of Enterobacteriaceae bacteria of plural genera in the sample include devices of the lateral-flow type and those of the flow-through type. According to the lateral-flow devices, the analyte to be detected and the antibody to be detected are deployed parallel onto a membrane having a detection area on the surface of which the capture antibody is immobilized, and the target substance captured in the detection area of the membrane is detected. On the other hand, according to the flow-through devices, the analyte to be detected and the detection antibody are passed vertically through a membrane on which the capture antibody is immobilized on the surface, and the target substance captured on the membrane surface is detected. The method of the present invention can be applied to both lateral-flow devices and flow-through devices.

Both lateral-flow type and flow-through type immunochromatographic detection devices are well known, and the details of such devices can be designed by those skilled in the art based on their technical knowledge, other than those described herein. The following is a description of the schematic configuration of the detection mechanism of the lateral flow type immunochromatographic detection device, with reference to the drawings. The schematic configuration of the detection mechanism of the lateral-flow type immunochromatography detection system will be described below with reference to the figure. However, these are only examples of the schematic configuration of the detection procedure, and the configuration of the lateral-flow type immunochromatographic detection system is not limited in any way to the embodiment illustrated in the figure.

Figure 1 is a cross-sectional view of a schematic configuration of a strip-shaped detection mechanism, which is an example of a detection mechanism of a lateral-flow type immunochromatographic detection system. The detection mechanism 10 of Figure 1 contains an insoluble membrane carrier 1 for chromatographic development; a strip-shaped member (conjugate pad) 2 (which is impregnated with the detection antibody) and a sample addition member (sample pad) 3 arranged at one end of the lengthwise direction of the strip (upstream of the sample flow B) on the insoluble membrane carrier 1; and a member for absorption (absorption pad) 4 arranged at the other end (downstream side of the specimen flow B) at the other end of the lengthwise direction of the strip (downstream of the sample flow B) on the insoluble membrane carrier 1. Arranged in the center of the strip lengthwise direction on the insoluble membrane carrier 1 is a site 5 on which the capture antibody is immobilized, along with, if necessary, a site 6 on which the control reagent is immobilized. The control reagent is a reagent that does not bind to the analyte but does bind to the detection antibody. When a sample A is applied on the sample addition member (sample pad) 3, the sample A passes through the member (conjugate pad) 2 impregnated with the detection antibody, and flows through the insoluble membrane carrier 1 in the sample flow direction A. During this process, the analyte in the sample (in the case of the present invention, Enterobacteriaceae bacteria to be detected) binds to the detection antibody to form an analyte-detection antibody complex. When the sample A passes through the capture-antibody immobilized site 5, the analyte in the sample binds to the capture antibody to form a capture antibody-analyte-detection antibody complex. When the sample A passes through the control-reagent immobilized site 6, the detection antibody that has not bound to the analyte binds to the control reagent 6, whereby it is confirmed that the test has been completed (i.e., that specimen A has passed through the capture antibody 5). The presence or amount of the analyte can be detected by detecting the label of the detection antibody in the capture antibody-analyte-detection antibody complex that exists in the capture-antibody immobilized site 5 by known means. If necessary, the label of the detection antibody may be sensitized by known methods to facilitate detection.

The types of the solid-phase carrier used for the capture antibody are not restricted, but specific examples include: porous membranes made of cellulose, nitrocellulose, cellulose acetate, nylon, PVDF (PolyVinylidene DiFluoride), glass fiber, etc.; flow channels made of glass, plastic, PDMS (Poly(dimethylsiloxane)), silicone, etc.; thread, paper, fiber, etc.

The method for attaching an antibody to the solid-phase carrier is also not restricted, but specific examples include fixation via physisorption using the hydrophobicity of the antibody and fixation via chemical bonding using a functional group of the antibody.

The type of detection label used for the detection antibody is not restricted and may be selected as appropriate in accordance with the detection method. Specific examples include: metal colloids such as gold colloids, platinum colloids, and palladium colloids; non-metal colloids such as selenium colloids, alumina colloids, and silica colloids; insoluble granular materials such as colored resin particles, dye colloids, colored liposomes, etc.; enzymes that catalyze chromogenic reactions such as alkaline phosphatase, peroxidase, luciferase, etc.; fluorescent dyes, radioisotopes; and chemiluminescent labels, bioluminescent labels, electrochemiluminescent labels, etc.

The method for attaching the label to the antibody is also not restricted, but specific examples of methods that can be used include physical adsorption using the hydrophobicity of the antibody, chemisorption using a functional group of the antibody, etc.

The detection antibody-impregnated member (conjugate pad) 2 and the sample addition member (sample pad) 3 may be optionally omitted. When the present mechanism lacks the detection antibody-impregnated member (conjugate pad) 2, the same test as above can be performed by applying the sample A and the detection antibody to one end on the insoluble membrane carrier 1 in a pre-mixed state or separately, either simultaneously or sequentially.

Even when the capture antibody and the detection antibody are interchanged, a detection kit can be constructed to enable the same detection. That is, the capture antibody may be the generic antibody and the detection antibody may be the specific antibody. Alternatively, the detection antibody may be the generic antibody and the capture antibody may be the specific antibody. In either case, either or both of the generic antibody and the specific antibody may be the antibody of the present invention, as described above.

According to the immunochromatographic detection method and device of the lateral-flow type, the two or more genera of Enterobacteriaceae bacteria as the target for detection can be detected at once on a single detection line (in the example shown in Figure 1, the capture-antibody immobilized site 5). Specifically, in the case of the conventional immunochromatographic detection method and device of the lateral-flow type, when two or more detection targets are detected, a detection line (in the example shown in Figure 1, the capture-antibody immobilized site 5) must be provided for each detection target, so that detection lines corresponding to the number of detection targets must be formed. This has resulted in the enlargement of the device, and when there are too many targets to be detected, it is difficult or impossible to detect them with a single device. On the other hand, in the case of the immunochromatographic detection method and device of the lateral-flow type, it is possible to simultaneously detect plural genera of Enterobacteriaceae bacteria on a single detection line, by combining the detection antibody and the capture antibody as appropriate. This enables downsizing of the device, and even when many species and genera of Enterobacteriaceae bacteria have to be detected, detection can be made with a single device, by determining the total amount of the bacteria to be detected on a single detection line.

Disclosed but not claimed is a method for producing the above-mentioned immunochromatography kit. The method includes at least the steps of stacking the conjugate pad, to which the detection antibody is attached, on the insoluble membrane carrier, and immobilizing the capture antibody in the chromatographic development direction opposite to the conjugate pad on the insoluble membrane carrier.

Either the capture antibody or the detection antibody may be used as the specific antibody, and either may be used as the generic antibody. However, it is preferable to use the specific antibody as the capture antibody and the generic antibody as the detection antibody.

The specific antibody may preferably be an antibody that causes antigen-antibody reactions specifically with at least Enterobacteriaceae bacteria of two or more genera selected from the genus Escherichia, the genus Klebsiella, the genus Citrobacter, the genus Enterobacter, the genus Proteus, the genus Salmonella, and the genus Serratia.

The generic antibody may preferably be an antibody that causes antigen-antibody reactions specifically with at least 5 or more genera selected from the genus Escherichia, the genus Klebsiella, the genus Citrobacter, the genus Enterobacter, the genus Proteus, the genus Salmonella, the genus Serratia, the genus Pseudomonas, the genus Staphylococcus, the genus Bacillus, and the genus Enterococcus.

According to the method for producing an immunochromatography kit, it is possible to use the same generic antibody as the detection antibody for mass production of plural variations of immunochromatography kits, which is extremely effective in that plural types of immunochromatography kits can be manufactured by selecting only the capture antibody.

### EXAMPLES

The present invention will be described in more detail with reference to the examples below. However, the present invention is not bound by the following examples, and can be implemented in any form within the scope that does not depart from the purpose of the present invention.

### [1. Production of Antibodies]

### *Production of Specific Antibodies A to C:

E. coli (EC) was used as an immunogen Enterobacteriaceae bacterium. Antibodies against ribosome protein L7/L12 of E. coli were produced by referring to the method described in WO2000/06603 A. Specifically, E. coli transformed with an expression vector incorporating DNA encoding the entire amino acid sequence of ribosomal protein L7/L12 of E. coli was cultured using, e.g., LB medium, and the ribosomal protein L7/L12 was purified by affinity column as a fusion protein using a tag sequence derived from the expression vector. The total length protein of E. coli L7/L12 was prepared as an immunogen in PBS according to the standard method for obtaining hybridomas, so that the concentration of the immunogen was 0.4 mg/mL, and Freund's adjuvant was added in the same volume. Mice were immunized four times with an immunogen level of 50 µg/time. After confirming the increase in serum antibody titer by test blood collection, mouse spleen cells were harvested. The excised mouse spleen cells were fused with myeloma cells, and various hybridomas were obtained.

The various hybridomas obtained were cultured in HAT medium, and screened based on antibodies in the culture supernatant. The screening was performed by ELISA using solid-phase lysates of several genera of Enterobacteriaceae as antigens, to select hybridomas producing antibodies simultaneously reactive with lysates of seven species of Enterobacteriaceae bacteria: Escherichia coli (EC), Klebsiella pneumoniae (KP), Citrobacter freundii (CF), Enterobacter cloacae (ECL), Proteus mirabilis (PM), Salmonella enterica (SE), and Serratia liquefaciens (SL). According to the established method of monoclonal antibody production, the selected hybridomas were cultured in TIL MediaI medium supplemented with 10% fetal bovine serum (FBS), injected into the abdominal cavity of a mouse, and ascites was collected. Antibodies were purified from the collected ascites by centrifuging the ascites to remove suspended solids and erythrocytes, filtering the supernatant through a filter with a mesh size of 0.45 µm, and pass the filtrate through a Protein G column to adsorb antibodies.

Through the above procedure, the specific antibodies A (EC50C1), B (EC149C3), and C (EC162A3) were obtained.

### *Production of Generic Antibodies A and B:

Pseudomonas aeruginosa (PA) was used as an immunogen bacterium. An antibody against ribosome protein L7/L12 of Pseudomonas aeruginosa was produced by referring to the method described in WO2000/06603 A. Specifically, E. coli transformed with an expression vector incorporating DNA encoding the entire amino acid sequence of ribosomal protein L7/L12 of Pseudomonas aeruginosa was cultured using, e.g., LB medium, and the ribosomal protein L7/L12 was purified by affinity column as a fusion protein using a tag sequence derived from the expression vector. The total length protein of Pseudomonas aeruginosa L7/L12 was prepared as an immunogen in PBS according to the standard method for obtaining hybridomas, so that the concentration of the immunogen was 0.4 mg/mL, and Freund's adjuvant was added in the same volume. Mice were immunized four times with an immunogen level of 50 µg/time. After confirming the increase in serum antibody titer by test blood collection, mouse spleen cells were harvested. The excised mouse spleen cells were fused with myeloma cells, and various hybridomas were obtained.

The various hybridomas obtained were cultured in HAT medium, and screened based on antibodies in the culture supernatant. The screening was performed by ELISA using solid-phase lysates of several genera of bacteria including non-Enterobacteriaceae bacteria as antigens, to select hybridomas producing antibodies simultaneously reactive with lysates of these several genera of bacteria. According to the established method of monoclonal antibody production, the selected hybridomas were cultured in TIL MediaI medium supplemented with 10% fetal bovine serum (FBS), injected into the abdominal cavity of a mouse, and ascites was collected. An antibody was purified from the collected ascites by centrifuging the ascites to remove suspended solids and erythrocytes, filtering the supernatant through a filter with a mesh size of 0.45 µm, and pass the filtrate through a Protein G column to adsorb the antibody, to thereby produce a generic antibody A (PA51B2).

The same procedure as that for producing the generic antibody A was carried out except that ribosome protein L7/L12 of Chlamydia pneumoniae (LP) was used as an immunogen, to thereby produce a generic antibody B (CP141A190.1).

### *Amino Acid Sequencing of Each Variable Region of the Heavy and Light Chains of Specific Antibodies A to C and Generic Antibodies A and B:

The amino acid sequences of each variable sequence of the heavy and light chains for the specific antibodies A to C (antibodies of the invention) and the generic antibodies A and B produced by the above procedure were determined according to the usual method. The correspondence between the amino acid sequences and the sequence identification numbers is shown below.

*Specific antibody A (EC50C1):
   Amino acid sequence of the heavy chain variable region (SEQ ID NO:1)
   Amino acid sequence of the light chain variable region (SEQ ID NO:2)
*Specific antibody B (EC149C3):
   Amino acid sequence of the heavy chain variable region (SEQ ID NO:3)
   Amino acid sequence of the light chain variable region (SEQ ID NO:4)
*Specific antibody C (EC162A3):
   Amino acid sequence of the heavy chain variable region (SEQ ID NO:5)
   Amino acid sequence of the light chain variable region (SEQ ID NO:6)
*Generic antibody A (PA51B2):
   Amino acid sequence of the heavy chain variable region (SEQ ID NO:7)
   Amino acid sequence of the light chain variable region (SEQ ID NO:8)
*Generic antibody B (CP141A190.1):
   Amino acid sequence of the heavy chain variable region (SEQ ID NO:9)
   Amino acid sequence of the light chain variable region (SEQ ID NO:10)

### [2. Detection of Antigen-Antibody Reactions between Antibodies and Enterobacteriaceae Bacteria 1 - Lysis Antigen Solid-Phase ELISA]

The thus-produced Specific Antibodies A to C (antibodies of the present invention) and Generic Antibodies A and B were used to obtain data on the reactivity with plural genera of Enterobacteriaceae bacteria and bacteria other than Enterobacteriaceae (non-Enterobacteriaceae bacteria) by means of ELISA. Seven species of Enterobacteriaceae bacteria were selected for the study: Escherichia coli (EC), Klebsiella pneumoniae (KP), Citrobacter freundii (CF), Enterobacter cloacae (ECL), Proteus mirabilis (PM), Salmonella enterica (SE), and Serratia liquefaciens (SL), which are frequently detected in food, beverage, environmental, or biological samples. Four species of bacteria other than Enterobacteriaceae (non-Enterobacteriaceae bacteria) were also selected for the study: Pseudomonas aeruginosa (PA), Staphylococcus aureus (SA), Bacillus subtilis (BS), and Enterococcus faecalis (EF), which are frequently detected in food, beverage, environmental, or biological samples. The seven species of Enterobacteriaceae bacteria and four species of non-Enterobacteriaceae bacteria described above were purchased and cultured from ATCC, prepared at 1 x e⁸ cfu/mL each, and suspended in PBS. Each bacterium was lysed by sonication, and debris was removed by filtration through a filter with a 0.45-µm mesh opening to obtain the bacterial lysate of each bacterium.

50 µL of the above bacterial lysate was dropped into each well of a 96-well polystyrene plate for ELISA and solidified on the bottom of the plate. After washing each well three times with PBS-T (PBS with Tween 20), blocking treatment was performed with PBS containing 1% BSA (Bovine Serum Albumin). After blocking, the wells were washed three times with PBS-T, and 50 µL of 10 µg/mL solution of each of the Specific Antibodies A to C and Generic Antibodies A and B was dropped into each well for 1 hour for antigen-antibody reactions. After the reactions, the samples were washed three times with PBS-T, and then 0.5 µg/mL of HRP (Horse Radish Peroxidase) labeled secondary antibody (Goat Anti-mouse IgG: Goat Anti-mouse IgG), which is the enzyme for detection, was added dropwise. After the reaction, the wells were washed five times with PBS-T, and 100 µL of a mixture of tetramethylbenzidine (TMB) and hydrogen peroxide, the chromogenic substrate, was added dropwise to each well for the chromogenic reaction. After 10 minutes, hydrochloric acid, the reaction stopping solution, was dropped into each well, and the absorbance of each well at 450 nm was measured with a plate reader.

The measurement results are shown in Table 1 below. The results shown in this table indicate that the Specific Antibodies A to C reacted more sensitively (with an absorbance of 0.3 or more for each bacterium) with the samples of seven Enterobacteriaceae species, compared to the sample without bacteria, and did not react (with an absorbance of less than 0.3 for each bacterium) with the samples of four non-Enterobacteriaceae species. In other words, it was confirmed that the Specific Antibodies A to C can selectively cause (detect) antigen-antibody reactions with the Enterobacteriaceae bacteria. On the other hand, the Generic Antibodies A and B showed antigen-antibody reactivity with all Enterobacteriaceae and non-Enterobacteriaceae bacteria.

### [Table 1]

**Table 1**

| | Negative Control | Enterobacteriaceae bacteria | | | | | | | Major bacteria in food/beverage/environment other than Enterobacteriaceae | | | |
|---|---|---|---|---|---|---|---|---|---|---|---|---|
| | Without Bacteria | EC | KP | CF | ECL | PM | SE | SL | PA | SA | BS | EF |
| Specific Antibody A | - | + | + | + | + | + | + | + | - | - | - | - |
| Specific Antibody B | - | + | + | + | + | + | + | + | - | - | - | - |
| Specific Antibody C | - | + | + | + | + | + | + | + | - | - | - | - |
| Generic Antibody A | - | + | + | + | + | + | + | + | + | + | + | + |
| Generic Antibody B | - | + | + | + | + | + | + | + | + | + | + | + |

| | | | | | | | | | | | | |
|---|---|---|---|---|---|---|---|---|---|---|---|---|
| -: Absorbance < 0.3 +: Absorbance ≥ 0.3 | | | | | | | | | | | | |

### [3. Detection of Antigen-Antibody Reactions between Antibodies and Enterobacteriaceae Bacteria 2 - Recombinant Antigen Solid-Phase ELISA]

The thus-produced Specific Antibodies A to C (antibodies of the present invention) and Generic Antibodies A and B were used to obtain data on the reactivity with ribosome protein L7/L12 of plural genera of Enterobacteriaceae bacteria by means of ELISA. Seven species of Enterobacteriaceae bacteria were selected for the study: Escherichia coli (EC), Klebsiella pneumoniae (KP), Citrobacter freundii (CF), Enterobacter cloacae (ECL), Proteus mirabilis (PM), Salmonella enterica (SE), and Serratia liquefaciens (SL), which are frequently detected in food, beverage, environmental, or biological samples. Four species of bacteria other than Enterobacteriaceae (non-Enterobacteriaceae bacteria) were also selected for the study: Pseudomonas aeruginosa (PA), Staphylococcus aureus (SA), Bacillus subtilis (BS), and Enterococcus faecalis (EF), which are frequently detected in food, beverage, environmental, or biological samples. E. coli transformed with expression vectors incorporating DNA encoding the amino acid sequence of ribosomal protein L7/L12 of each of the above seven Enterobacteriaceae and four non-Enterobacteriaceae species were cultured using, e.g., LB medium, and the ribosomal protein L7/L12 was purified as a fusion protein by affinity columns using tag sequences derived from the expression vector.

50 µL of 10 ng/mL solution of the ribosomal protein L7/L12 of each bacterial species was dropped into each well of a 96-well polystyrene plate for ELISA and solidified on the bottom of the plate. After washing each well three times with PBS-T (PBS with Tween 20), blocking treatment was performed with PBS containing 1% BSA (Bovine Serum Albumin). After blocking, the wells were washed three times with PBS-T, and 50 µL of 10 µg/mL solution of each of the Specific Antibodies A to C and Generic Antibodies A and B was dropped into each well for 1 hour for antigen-antibody reactions. After the reactions, the samples were washed three times with PBS-T, and then 0.5 µg/mL of HRP (Horse Radish Peroxidase) labeled secondary antibody (Goat Anti-mouse IgG: Goat Anti-mouse IgG), which is the enzyme for detection, was added dropwise. After the reaction, the wells were washed five times with PBS-T, and 100 µL of a mixture of tetramethylbenzidine (TMB) and hydrogen peroxide, the chromogenic substrate, was added dropwise to each well for the chromogenic reaction. After 10 minutes, hydrochloric acid, the reaction stopping solution, was dropped into each well, and the absorbance of each well at 450 nm was measured with a plate reader.

The measurement results are shown in Table 2 below. The results shown in this table indicate that the Specific Antibodies A to C reacted more sensitively (with an absorbance of 0.3 or more for each bacterium) with the samples of seven Enterobacteriaceae species, compared to the sample without bacteria, and did not react (with an absorbance of less than 0.3 for each bacterium) with the samples of four non-Enterobacteriaceae species. In other words, it was confirmed that the Specific Antibodies A to C can selectively cause (detect) antigen-antibody reactions with the ribosomal protein L7/L12 of each Enterobacteriaceae bacteria. On the other hand, the Generic Antibodies A and B showed antigen-antibody reactivity with all Enterobacteriaceae and non-Enterobacteriaceae bacteria.

### [Table 2]

**Table 2**

| | Negative Control | Enterobacteriaceae bacteria | | | | | | | Major bacteria in food/beverage/environment other than Enterobacteriaceae | | | |
|---|---|---|---|---|---|---|---|---|---|---|---|---|
| | Without Bacteria | EC | KP | CF | ECL | PM | SE | SL | PA | SA | BS | EF |
| Specific Antibody A | - | + | + | + | + | + | + | + | - | - | - | - |
| Specific Antibody B | - | + | + | + | + | + | + | + | - | - | - | - |
| Specific Antibody C | - | + | + | + | + | + | + | + | - | - | - | - |
| Generic Antibody A | - | + | + | + | + | + | + | + | + | + | + | + |
| Generic Antibody B | - | + | + | + | + | + | + | + | + | + | + | + |

| | | | | | | | | | | | | |
|---|---|---|---|---|---|---|---|---|---|---|---|---|
| -: Absorbance < 0.3 +: Absorbance ≥ 0.3 | | | | | | | | | | | | |

### [4. Verification of Cross-Reactivity between Antibodies and Non-Enterobacterial Components in Food/Beverage/Environmental Samples]

The thus-produced Specific Antibodies A to C (antibodies of the present invention) and Generic Antibodies A and B were used to obtain data on the reactivity with non-bacterial components in various food, beverage, or environmental samples (food, beverage, or environmental components) by means of ELISA. Raw fish (yellowtail and horse mackerel), raw noodles (yakisoba), raw egg, prepared food (potato salad), vegetables (cucumber (fruit vegetable), carrot (root vegetable), and lettuce (leaf vegetable)), and meats and processed meats (beef ribs, beef short ribs, pork loin, chicken breast, and ham) were purchased at a supermarket and used as food samples. Each of these foodstuffs was weighed 25 g, placed in a commercial stomacher bag, and stomached with 225 ml of PBS. A portion of the stomacher-treated solution was processed through a filter with a mesh aperture of 0.45 µm to remove solids containing bacteria, thereby preparing a bacteria-free food sample for ELISA. Milk and tea were purchased at a supermarket and used as beverage samples. Each of these beverages was suspended in PBS to a concentration of 1/10, and solids containing bacteria were removed with a filter with a 0.45 µm mesh opening, thereby preparing a bacteria-free beverage sample for ELISA. As environmental samples, hand fingers, cutting boards, kitchen knives, and refrigerator handles were wiped with a commercially available wiping kit (ELMEX Pro-mediaST-25, PBS), suspended in PBS provided with the kit, and solids containing bacteria were removed with a filter with a 0.45 µm mesh opening, thereby preparing a bacteria-free environmental sample for ELISA.

50 µL of each of the food, beverage, and environment samples was dropped into each well of a 96-well polystyrene plate for ELISA and solidified on the bottom of the plate. After washing each well three times with PBS-T (PBS with Tween 20), blocking treatment was performed with PBS containing 1% BSA. After blocking, the wells were washed three times with PBS-T, and 50 µL of 10 µg/mL solution of each of the Specific Antibodies A to C and Generic Antibodies A and B was dropped into each well for 1 hour for reactions. After the reactions, the samples were washed three times with PBS-T, and then 0.5 µg/mL of HRP (Horse Radish Peroxidase) labeled secondary antibody (Goat Anti-mouse IgG: Goat Anti-mouse IgG), which is the enzyme for detection, was added dropwise. After the reaction, the wells were washed five times with PBS-T, and 100 µL of a mixture of TMB and hydrogen peroxide, the chromogenic substrate, was added dropwise to each well for the chromogenic reaction. After 10 minutes, hydrochloric acid, the reaction stopping solution, was dropped into each well, and the absorbance of each well at 450 nm was measured with a plate reader.

The measurement results are shown in Table 3 below. The results shown in this table indicate that the Specific Antibodies A to C did not react with any of the above non-bacterial components in the food, beverage, or environmental samples (with an absorbance of less than 0.3 for each sample). Combined with the results of the aforementioned examples, it was confirmed that all of the Specific Antibodies A to C do not react with any of the non-bacterial components in the food, beverage, or environmental samples (food, beverage, or environmental components), but only with the specific plural genera of Enterobacteriaceae bacteria to be detected. In other words, it was confirmed that these antibodies can detect with high selectivity the presence and/or amount of the specific plural genera of Enterobacteriaceae bacteria to be detected in the food, beverage, or environmental samples. On the other hand, the Generic Antibodies A and B did not show cross-reactivity with any of the non-Enterobacterial components in the food, beverage, or environmental samples.

**[Table 3-1]**

| Table 3 | | | | | | | |
|---|---|---|---|---|---|---|---|
| | Food or beverage samples | | | | | | |
| | Raw fish | Raw noodles | Raw egg | Prepared food | Vegetables | Meat | Beverage |
| Specific Antibody A | - | - | - | - | - | - | - |
| Specific Antibody B | - | - | - | - | - | - | - |
| Specific Antibody C | - | - | - | - | - | - | - |
| Generic Antibody A | - | - | - | - | - | - | - |
| Generic Antibody B | - | - | - | - | - | - | - |

| | | | | | | | |
|---|---|---|---|---|---|---|---|
| -: Absorbance < 0.3 +: Absorbance ≥ 0.3 | | | | | | | |

**[Table 3-2]**

| Table 3 (continued) | | | | |
|---|---|---|---|---|
| | Environmental samples | | | |
| | Hand fingers | Cutting board | Kitchen knife | Refrigerator handle |
| Specific Antibody A | - | - | - | - |
| Specific Antibody B | - | - | - | - |
| Specific Antibody C | - | - | - | - |
| Generic Antibody A | - | - | - | - |
| Generic Antibody B | - | - | - | - |

| | | | | |
|---|---|---|---|---|
| -: Absorbance < 0.3 +: Absorbance ≥ 0.3 | | | | |

### [5. Production of Immunochromatographic Detection Kits]

Three types of immunochromatographic detection kits (a) to (c) were prepared by using the Specific Antibodies A to C (antibodies of the present invention) as primary antibodies (capture antibodies) and the Generic Antibodies A and B as secondary antibodies (detection antibodies) in the combinations shown in (a) to (c) of Table 4 below. In addition, additional three types of immunochromatographic detection kits (d) to (f) capable of similar detection were prepared by changing the antibodies used for the primary and secondary antibodies in the combinations listed in (d) to (f) of Table 4 below.

### [Table 4]

**Table 4**

| Immunochromatography detection kit | Bacteria to be detected | Primary antibody (capture antibody) | Secondary antibody (detection antibody) |
|---|---|---|---|
| (a) | Enterobacteriaceae bacteria | Specific Antibody A (EC50C1) | Generic Antibody A (PA51B2) |
| (b) | Enterobacteriaceae bacteria | Specific Antibody A (EC50C1) | Generic Antibody B (CP141A190.1) |
| (c) | Enterobacteriaceae bacteria | Specific Antibody B (EC149C3) | Generic Antibody B (CP141A190.1) |
| (d) | Enterobacteriaceae bacteria | Generic Antibody A (PA51B2) | Specific Antibody A (EC50C1) |
| (e) | Enterobacteriaceae bacteria | Generic Antibody B (CP141A190.1) | Specific Antibody A (EC50C1) |
| (f) | Enterobacteriaceae bacteria | Generic Antibody B (CP141A190.1) | Specific Antibody B (EC149C3) |

### *Production of Membrane Carrier for Immunochromatography Development:

A solution was prepared that contain 1.5 mg/mL of each primary antibody (capture antibody) as listed in (a) to (f) of Table 4 above and 3% (v/v) of trehalose in 10 mM sodium phosphate buffer solution. The resulting solution was applied to a commercially available nitrocellulose membrane cut to 2.5 cm wide and 15 cm long at a volume of 1 µL solution per cm² and dried to make a membrane carrier for immunochromatography development.

### *Preparation of Antibodies for Gold Colloid Labeling Detection and Antibody-Impregnated Members for Gold Colloid Labeling Detection:

A solution with an antibody concentration of 0.1 mg/mL was prepared by mixing a commercially available gold colloid solution (60 nm particle size) with 1/10 volume of each secondary antibody (detection antibody) listed in (a) to (f) of Table 4 above. The solution was allowed to stand at room temperature for 30 minutes to allow the antibody to bind to the surface of the gold colloidal particles. The antibody solution was then blocked by adding BSA solution so that the final concentration in the gold colloid solution was 0.1%, whereby the antibody solution for gold colloid labeling detection was prepared. This antibody solution was soaked into commercially available glass fiber sheets and dried to prepare an antibody-impregnated member for gold colloid labeling detection.

### *Assembly of Immunochromatographic Detection Kits:

In addition to the membrane carrier for immunochromatographic development and the antibody-impregnated member for gold colloid labeling detection prepared by the procedures described above, a cotton cloth for adding a sample and a filter paper for absorbing a sample were also prepared for each kit. These components were laminated onto a commercially available polyethylene substrate, cut into 5 mm widths, to prepare an immunochromatographic detection kit with a detection mechanism as shown in Figure 1.

### [6. Detection of Bacteria using the Immunochromatographic Detection Kits]

The thus-obtained immunochromatographic detection kits (a) to (f) were used to detect plural genera of Enterobacteriaceae bacteria. Seven species of Enterobacteriaceae bacteria were selected for the study: Escherichia coli (EC), Klebsiella pneumoniae (KP), Citrobacter freundii (CF), Enterobacter cloacae (ECL), Proteus mirabilis (PM), Salmonella enterica (SE), and Serratia liquefaciens (SL), which are frequently detected in food, beverage, environmental, or biological samples. Four species of bacteria other than Enterobacteriaceae (non-Enterobacteriaceae bacteria) were also selected for the study: Pseudomonas aeruginosa (PA), Staphylococcus aureus (SA), Bacillus subtilis (BS), and Enterococcus faecalis (EF), which are frequently detected in food, beverage, environmental, or biological samples. The seven species of Enterobacteriaceae bacteria and the four species of non-Enterobacteriaceae bacteria were each prepared at 1 × e⁸ cfu/mL and suspended in PBS. Each bacterium was lysed by sonication to obtain bacterial lysates (bacterial samples for immunochromatographic assay) of the seven Enterobacteriaceae bacteria and the four non-Enterobacteriaceae bacteria. A PBS solution without bacterial lysates was also prepared as a bacteria-free sample. Tween 20 was added to each of these samples at a final concentration of 1%, whereby samples for immunochromatographic development were prepared.

The prepared samples (one bacteria-free sample and 11 bacterial lysate samples) were each added to the sample addition member area of each of the immunochromatographic detection kits having the configurations of (a) through (f) above, and 30 minutes later, the line coloration of the capture-antibody applied area of the membrane carrier was visually confirmed.

The results of the detection kits (a) through (c) are shown in Table 5 below. These results indicate that as intended, line coloration was confirmed for all Enterobacteriaceae bacteria in all of the detection kits (a) through (c), indicating that antigen-antibody reactions occurred. On the other hand, no line coloration was confirmed for the bacteria other than Enterobacteriaceae bacteria (non-Enterobacteriaceae bacteria) in all of the detection kits (a) through (c), indicating that no antigen-antibody reactions occurred. In other words, each immunochromatographic detection kit constructed by combining a specific antibody, that causes antigen-antibody reactions only with Enterobacteriaceae bacteria, and a generic antibody, that causes antigen-antibody reactions widely with bacteria of many genera, was shown to be able to detect the Enterobacteriaceae bacteria easily and rapidly. The detection kits using combinations of (d) through (f) in Table 4 above were also tested for detection of the plural genera of Enterobacteriaceae, and similar results were obtained.

### [Table 5]

**Table 5**

| Detection kit | Negative control | Enterobacteriaceae bacteria | | | | | | | Major bacteria in food/beverage/environment other than Enterobacteriaceae | | | |
|---|---|---|---|---|---|---|---|---|---|---|---|---|
| No. | Without bacteria | EC | KP | CF | ECL | PM | SE | SL | PA | SA | BS | EF |
| (a) | - | + | + | + | + | + | + | + | - | - | - | - |
| (b) | - | + | + | + | + | + | + | + | - | - | - | - |
| (c) | - | + | + | + | + | + | + | + | - | - | - | - |

| | | | | | | | | | | | | |
|---|---|---|---|---|---|---|---|---|---|---|---|---|
| -: Visually negative +: Visually positive | | | | | | | | | | | | |

### [7. Verification of Cross-Reactivity between Antibodies and Components in Food/Beverage/Environmental Samples using Immunochromatographic Detection Kit]

The thus-obtained immunochromatographic detection kits (a) to (f) were used to obtain data on the reactivity with non-bacterial components in various food, beverage, or environmental samples (food, beverage, or environmental components). Raw fish (yellowtail and horse mackerel), raw noodles (yakisoba), raw egg, prepared food (potato salad), vegetables (cucumber (fruit vegetable), carrot (root vegetable), and lettuce (leaf vegetable)), and meats and processed meats (beef ribs, beef short ribs, pork loin, chicken breast, and ham) were purchased at a supermarket and used as food samples. Each of these foodstuffs was weighed 25 g, placed in a commercial stomacher bag, and stomached with 225 ml of PBS. A portion of the stomacher-treated solution was processed through a filter with a mesh aperture of 0.45 µm to remove solids containing bacteria, thereby preparing a sample for immunochromatography. Milk and tea were purchased at a supermarket and used as beverage samples. Each of these beverages was suspended in PBS to a concentration of 1/10, and solids containing bacteria were removed with a filter with a 0.45 µm mesh opening, thereby preparing a sample for immunochromatography. As environmental samples, hand fingers, cutting boards, kitchen knives, and refrigerator handles were wiped with a commercially available wiping kit (ELMEX Pro-mediaST-25, PBS), suspended in PBS provided with the kit, and solids containing bacteria were removed with a filter with a 0.45 µm mesh opening, thereby preparing a sample for immunochromatography.

The samples prepared for immunochromatography from various food, beverage, or environmental samples were each added to the sample addition member area of each of the immunochromatographic detection kits having the configurations of (a) through (f) above, and 30 minutes later, the line coloration of the capture-antibody applied area of the membrane carrier was visually confirmed. The results for the detection kits (a) through (c) are shown in Table 6. As intended, none of the detection kits (a) through (c) showed cross-reactivity with the components in the above food, beverage, or environmental samples. In combination with the aforementioned examples, it was confirmed that the immunochromatographic detection kits (a) through (c) prepared by combining the aforementioned generic and specific antibodies do not react with non-bacterial components (food, beverage, or environmental components) in the food, beverage, or environmental samples, and makes it possible to detect only the specific Enterobacteriaceae bacteria to be detected easily and rapidly with high selectivity. The detection kits using combinations of (d) through (f) in Table 4 above were also tested for cross-reactivity with non-bacterial components in the food, beverage, or environmental samples, and similar results were obtained.

**[Table 6-1]**

| Table 6 | | | | | | | |
|---|---|---|---|---|---|---|---|
| Detection kit | Food or beverage samples | | | | | | |
| No. | Raw fish | Raw noodles | Raw egg | Prepared food | Vegetables | Meat | Beverage |
| (a) | - | - | - | - | - | - | - |
| (b) | - | - | - | - | - | - | - |
| (c) | - | - | - | - | - | - | - |

| | | | | | | | |
|---|---|---|---|---|---|---|---|
| -: Visually negative +: Visually positive | | | | | | | |

**[Table 6-2]**

| Table 6 (continued) | | | | |
|---|---|---|---|---|
| Detection kit | Environmental samples | | | |
| No. | Hand fingers | Cutting board | Kitchen knife | Refrigerator handle |
| (a) | - | - | - | - |
| (b) | - | - | - | - |
| (c) | - | - | - | - |

| | | | | |
|---|---|---|---|---|
| -: Visually negative +: Visually positive | | | | |

### INDUSTRIAL APPLICABILITY

The present invention can be widely used in fields where simultaneous and simple detection of plural genera of Enterobacteriaceae bacteria in food, beverage, environmental, or biological samples is required, mainly in the fields of medicines and foods, and therefore has extremely high industrial usefulness.

### EXPLANATION OF SYMBOLS

- 10: Detection mechanism
- 1: Insoluble membrane carrier for chromatography development
- 2: Detection antibody-impregnated member (conjugate pad)
- 3: Sample addition member (sample pad)
- 4: Member for absorption (absorption pad)
- 5: Capture-antibody immobilized site
- 6: Control-reagent immobilized site
- A: Sample
- B: Sample flow

## Claims

1. A method for detecting the presence and/or amount of Enterobacteriaceae bacteria in a sample selected from food or beverage samples, environmental samples, and biological samples, the method comprising the step of simultaneously detecting the presence and/or amount of Enterobacteriaceae bacteria of two or more different genera in the sample based on antigen-antibody reactions,
wherein the step of detecting comprises:
contacting the sample with an antibody that causes antigen-antibody reactions with components derived from the Enterobacteriaceae bacteria of two or more genera; and
measuring the presence and/or intensity of the antigen-antibody reaction that occurs in the sample after contact,
wherein the antibody is a monoclonal antibody having:
as a heavy chain variable region sequence, the amino acid sequence having a homology of 80% or more to the amino acid sequence of SEQ ID NO:1, and as a light chain variable region sequence, the amino acid sequence having a homology of 80% or more to the amino acid sequence of SEQ ID NO:2; or
as a heavy chain variable region sequence, the amino acid sequence having a homology of 80% or more to the amino acid sequence of SEQ ID NO:3, and as a light chain variable region sequence, the amino acid sequence having a homology of 80% or more to the amino acid sequence of SEQ ID NO:4; or
as a heavy chain variable region sequence, the amino acid sequence having a homology of 80% or more to the amino acid sequence of SEQ ID NO:5, and as a light chain variable region sequence, the amino acid sequence having a homology of 80% or more to the amino acid sequence of SEQ ID NO:6.

2. The method according to claim 1, wherein the step of detecting includes simultaneously detecting Enterobacteriaceae bacteria of two or more, or 3 or more, or 4 or more, or 5 or more, or 6 or more, or 7 or more different genera selected from the group consisting of the genus Escherichia, the genus Klebsiella, the genus Citrobacter, the genus Enterobacter, the genus Proteus, the genus Salmonella, and the genus Serratia.

3. The method according to claim 1 or 2, wherein the components are ribosome proteins L7/L12 of the Enterobacteriaceae bacteria of two or more genera.

4. The method according to claim 3, wherein the antibody does not cause cross-reactions with components derived from one or more non-Enterobacteriaceae bacteria that may be present in the sample,
optionally wherein the one or more non-Enterobacteriaceae bacteria are one or more bacteria selected from the genus Pseudomonas, the genus Staphylococcus, the genus Bacillus, and the genus Enterococcus.

5. The method according to claim 3 or 4, wherein the antibody does not cause cross-reactions with at least one non-bacterial component that may be present in the sample,
optionally wherein the non-bacterial component is an organic component derived from a virus, plant, and/or animal.

6. The method according to any one of claims 1 to 5, comprising detecting the presence and/or amount of Enterobacteriaceae bacteria in the sample by the steps of:
(I) capturing the bacteria in the sample and detecting the bacteria in the sample based on antigen-antibody reactions between the sample, a capture antibody bound to a solid-phase carrier, and a detection antibody having a detection label, and
(II) detecting the bacteria to be detected in the sample based on the detection label,
wherein one of the capture antibody and the detection antibody is at least one specific antibody, which causes antigen-antibody reactions with two or more species of bacteria to be detected, and the other is at least one generic antibody, which causes antigen-antibody reactions with bacteria of five or more genera including the bacteria to be detected, and
wherein either the generic antibody or the specific antibody is an antibody as recited in any one of claims 1 to 5.

7. The method according to claim 6, wherein step (I) includes the steps of:
(Ia-1) contacting the detection antibody with the sample to thereby detect bacteria in the sample based on the antigen-antibody reaction between the detection antibody and the bacteria; and
(Ia-2) contacting the capture antibody with the sample containing the bacteria labeled by the detection antibody to thereby capture the bacteria in the sample based on the antigen-antibody reaction between the capture antibody and the bacteria-detection antibody complex, or
wherein step (I) includes the steps of:
(Ib-1) contacting the capture antibody with the sample to thereby capture bacteria in the sample based on the antigen-antibody reaction between the capture antibody and the bacteria, and
(Ib-2) contacting the detection antibody with the sample containing the bacteria captured by the capture antibody to thereby detect the bacteria in the sample based on the antigen-antibody reaction between the detection antibody and the bacteria-capture antibody complex.

8. The method according to claim 6 or 7, wherein the capture antibody is the generic antibody, and the detection antibody is the specific antibody, or
wherein the detection antibody is the generic antibody, and the capture antibody is the specific antibody.

9. The method according to any one of claims 6 to 8, wherein the specific antibody causes antigen-antibody reactions with Enterobacteriaceae bacteria of two or more genera selected from at least the genus Escherichia, the genus Klebsiella, the genus Citrobacter, the genus Enterobacter, the genus Proteus, the genus Salmonella, and the genus Serratia, and/or
wherein the generic antibody causes antigen-antibody reactions with bacteria of five or more genera selected from at least the genus Escherichia, the genus Klebsiella, the genus Citrobacter, the genus Enterobacter, the genus Proteus, the genus Salmonella, the genus Serratia, the genus Pseudomonas, the genus Staphylococcus, the genus Bacillus, and the genus Enterococcus.

10. The method according to any one of claims 6 to 9, wherein the specific antibody comprises:
as a heavy chain variable region sequence, the amino acid sequence having a homology of 80% or more to the amino acid sequence of SEQ ID NO:1, and as a light chain variable region sequence, an amino acid sequence having a homology of 80% or more to the amino acid sequence of SEQID NO:2; or
as a heavy chain variable region sequence, the amino acid sequence having a homology of 80% or more to the amino acid sequence of SEQ ID NO:3, and as a light chain variable region sequence, an amino acid sequence having a homology of 80% or more to the amino acid sequence of SEQID NO:4; or
as a heavy chain variable region sequence, the amino acid sequence having a homology of 80% or more to the amino acid sequence of SEQ ID NO:5, and as a light chain variable region sequence, an amino acid sequence having a homology of 80% or more to the amino acid sequence of SEQ ID NO:6, and/or
wherein the generic antibody comprises:
as a heavy chain variable region sequence, the amino acid sequence having a homology of 80% or more to the amino acid sequence of SEQ ID NO:7, and as a light chain variable region sequence, an amino acid sequence having a homology of 80% or more to the amino acid sequence of SEQ ID NO:8; or
as a heavy chain variable region sequence, the amino acid sequence having a homology of 80% or more to the amino acid sequence of SEQ ID NO:9, and as a light chain variable region sequence, an amino acid sequence having a homology of 80% or more to the amino acid sequence of SEQ ID NO: 10.

11. A method for determining the degree of contamination by Enterobacteriaceae bacteria in a sample selected from food or beverage samples, environmental samples, and biological samples, comprising the step of simultaneously detecting the presence and/or amount of Enterobacteriaceae bacteria of two or more genera in the sample based on antigen-antibody reactions by the method according to any one of claims 1 to 10.

12. A kit for detecting the presence and/or amount of Enterobacteriaceae bacteria in a sample selected from food or beverage samples, environmental samples, and biological samples by the method according to any one of claims 1 to 5, comprising an antibody as recited in any one of claims 1 to 5.

13. A kit for detecting the presence and/or amount of Enterobacteriaceae bacteria in a sample selected from food or beverage samples, environmental samples, and biological samples by the method according to any one of claims 6 to 10, comprising a capture antibody and a detection antibody as recited in any one of claims 6 to 10, wherein one of the capture antibody and the detection antibody is the generic antibody and the other is the specific antibody,
optionally the kit further comprises an insoluble membrane carrier for developing the sample and contacting the sample with the capture antibody, wherein the insoluble membrane carrier has a detection line formed thereon and the capture antibody is immobilized on the detection line, whereby two or more species of bacteria in the sample are detected on the single detection line.

14. The kit according to claim 12 or 13, which is an immunochromatography kit, optionally wherein the capture antibody and the detection antibody are selected so as to cause antigen-antibody reactions with the ribosome protein L7/L12 of the Enterobacteriaceae bacteria to be detected in the sample to form a sandwich structure,
wherein the immunochromatography kit further comprises:
an insoluble membrane carrier for developing the sample and contacting the sample with the capture antibody; and
a conjugate pad which is formed on the insoluble membrane carrier and to which the detection antibody is attached, and
wherein the capture antibody is immobilized to the conjugate pad on the insoluble membrane carrier along the development direction of chromatography.

## Patentansprüche

1. Verfahren zum Nachweis des Vorhandenseins und/oder der Menge von Enterobacteriaceae-Bakterien in einer Probe, die aus Lebensmittel- oder Getränkeproben, Umweltproben und biologischen Proben ausgewählt ist, wobei das Verfahren den Schritt des gleichzeitigen Nachweises des Vorhandenseins und/oder der Menge von Enterobacteriaceae-Bakterien von zwei oder mehr verschiedenen Gattungen in der Probe auf der Grundlage von Antigen-Antikörper-Reaktionen umfasst,
wobei der Nachweis-Schritt umfasst:
Inkontaktbringen der Probe mit einem Antikörper, der Antigen-Antikörper-Reaktionen mit von Enterobacteriaceae-Bakterien zweier oder mehrerer Gattungen stammenden Komponenten hervorruft; und
Messen des Vorhandenseins und/oder der Intensität der Antigen-Antikörper-Reaktion, die in der Probe nach dem Kontakt auftritt,
wobei der Antikörper ein monoklonaler Antikörper ist, der Folgendes umfasst:
als Sequenz der variablen Region der schweren Kette weist die Aminosäuresequenz eine Homologie von 80 % oder mehr zur Aminosäuresequenz von SEQ ID NO:1 auf, und als Sequenz der variablen Region der leichten Kette weist die Aminosäuresequenz eine Homologie von 80 % oder mehr zur Aminosäuresequenz von SEQ ID NO:2 auf; oder
als Sequenz der variablen Region der schweren Kette weist die Aminosäuresequenz eine Homologie von 80 % oder mehr zur Aminosäuresequenz von SEQ ID NO:3 auf, und als Sequenz der variablen Region der leichten Kette weist die Aminosäuresequenz eine Homologie von 80 % oder mehr zu der Aminosäuresequenz von SEQ ID NO:4 auf; oder
als Sequenz der variablen Region der schweren Kette weist die Aminosäuresequenz eine Homologie von 80 % oder mehr zur Aminosäuresequenz von SEQ ID NO:5 auf, und als Sequenz der variablen Region der leichten Kette weist die Aminosäuresequenz eine Homologie von 80 % oder mehr zur Aminosäuresequenz von SEQ ID NO:6 auf.

2. Verfahren nach Anspruch 1, wobei der Nachweis-Schritt den gleichzeitigen Nachweis von Enterobacteriaceae-Bakterien von zwei oder mehr, oder 3 oder mehr, oder 4 oder mehr, oder 5 oder mehr, oder 6 oder mehr, oder 7 oder mehr verschiedenen Gattungen, ausgewählt aus der Gruppe bestehend aus der Gattung Escherichia, der Gattung Klebsiella, der Gattung Citrobacter, der Gattung Enterobacter, der Gattung Proteus, der Gattung Salmonella und der Gattung Serratia, beinhaltet.

3. Verfahren nach Anspruch 1 oder 2, wobei es sich bei den Komponenten um Ribosomenproteine L7/L12 der Enterobacteriaceae-Bakterien von zwei oder mehr Gattungen handelt.

4. Verfahren nach Anspruch 3, wobei der Antikörper keine Kreuzreaktionen mit Komponenten verursacht, die von einem oder mehreren Nicht-Enterobacteriaceae-Bakterien stammen, die in der Probe vorhanden sein können,
gegebenenfalls wobei das eine oder die mehreren Nicht-Enterobacteriaceae-Bakterien ein oder mehrere Bakterien sind, die aus der Gattung Pseudomonas, der Gattung Staphylococcus, der Gattung Bacillus und der Gattung Enterococcus ausgewählt sind.

5. Verfahren nach Anspruch 3 oder 4, wobei der Antikörper keine Kreuzreaktionen mit mindestens einer nicht-bakteriellen Komponente verursacht, die in der Probe vorhanden sein kann,
gegebenenfalls wobei die nichtbakterielle Komponente eine organische Komponente ist, die von einem Virus, einer Pflanze und/oder einem Tier stammt.

6. Verfahren nach einem der Ansprüche 1 bis 5, umfassend den Nachweis des Vorhandenseins und/oder der Menge von Enterobacteriaceae-Bakterien in der Probe durch die folgenden Schritte:
(I) Einfangen der Bakterien in der Probe und Nachweis der Bakterien in der Probe auf Grundlage von Antigen-Antikörper-Reaktionen zwischen der Probe, einem an einen Festphasenträger gebundenen Einfang-Antikörper und einem Nachweis-Antikörper mit einer Nachweis-Markierung, und
(II) Nachweis der in der Probe nachzuweisenden Bakterien auf der Grundlage des Nachweis-Markierung,
wobei einer der Einfang-Antikörper und der Nachweis-Antikörper mindestens ein spezifischer Antikörper ist, der Antigen-Antikörper-Reaktionen mit zwei oder mehr Arten von nachzuweisenden Bakterien verursacht, und der andere mindestens ein generischer Antikörper ist, der Antigen-Antikörper-Reaktionen mit Bakterien von fünf oder mehr Gattungen einschließlich der nachzuweisenden Bakterien verursacht, und
wobei entweder der generische Antikörper oder der spezifische Antikörper ein Antikörper ist, wie er in einem der Ansprüche 1 bis 5 angegeben ist.

7. Verfahren nach Anspruch 6, wobei Schritt (I) die folgenden Schritte umfasst:
(Ia-1) Inkontaktbringen des Nachweisantikörpers mit der Probe, um dadurch Bakterien in der Probe auf der Grundlage der Antigen-Antikörper-Reaktion zwischen dem Nachweisantikörper und den Bakterien nachzuweisen; und
(Ia-2) Inkontaktbringen des Einfang-Antikörpers mit der Probe, die die durch den Nachweis-Antikörper markierten Bakterien enthält, um dadurch die Bakterien in der Probe auf Grundlage der Antigen-Antikörper-Reaktion zwischen dem Einfang-Antikörper und dem Bakterien-Nachweis-Antikörper-Komplex einzufangen, oder
wobei Schritt (I) die folgenden Schritte umfasst:
(Ib-1) Inkontaktbringen des Einfang-Antikörpers mit der Probe, um dadurch Bakterien in der Probe auf Grundlage der Antigen-Antikörper-Reaktion zwischen dem Einfang-Antikörper und den Bakterien einzufangen, und
(Ib-2) Inkontaktbringen des Nachweis-Antikörpers mit der Probe, die die von dem Einfang-Antikörper eingefangenen Bakterien enthält, um dadurch die Bakterien in der Probe auf Grundlage der Antigen-Antikörper-Reaktion zwischen dem Nachweis-Antikörper und dem Bakterien-Einfang-Antikörper-Komplex nachzuweisen.

8. Verfahren nach Anspruch 6 oder 7, wobei der Einfang-Antikörper der generische Antikörper und der Nachweis-Antikörper der spezifische Antikörper ist, oder
wobei der Nachweis-Antikörper der generische Antikörper und der Einfang-Antikörper der spezifische Antikörper ist.

9. Verfahren nach einem der Ansprüche 6 bis 8, wobei der spezifische Antikörper Antigen-Antikörper-Reaktionen mit Enterobacteriaceae-Bakterien von zwei oder mehreren Gattungen, ausgewählt aus mindestens der Gattung Escherichia, der Gattung Klebsiella, der Gattung Citrobacter, der Gattung Enterobacter, der Gattung Proteus, der Gattung Salmonella und der Gattung Serratia, und/oder
wobei der generische Antikörper Antigen-Antikörper-Reaktionen mit Bakterien von fünf oder mehr Gattungen, ausgewählt aus mindestens der Gattung Escherichia, der Gattung Klebsiella, der Gattung Citrobacter, der Gattung Enterobacter, der Gattung Proteus, der Gattung Salmonella, der Gattung Serratia, der Gattung Pseudomonas, der Gattung Staphylococcus, der Gattung Bacillus und der Gattung Enterococcus, verursacht.

10. Verfahren nach einem der Ansprüche 6 bis 9, wobei der spezifische Antikörper umfasst:
als Sequenz der variablen Region der schweren Kette weist die Aminosäuresequenz eine Homologie von 80 % oder mehr zur Aminosäuresequenz von SEQ ID NO:1 auf, und als Sequenz der variablen Region der leichten Kette weist die Aminosäuresequenz eine Homologie von 80 % oder mehr zur Aminosäuresequenz von SEQ ID NO:2 auf; oder
als Sequenz der variablen Region der schweren Kette weist die Aminosäuresequenz eine Homologie von 80 % oder mehr zur Aminosäuresequenz von SEQ ID NO:3 auf, und als Sequenz der variablen Region der leichten Kette weist die Aminosäuresequenz eine Homologie von 80 % oder mehr zu der Aminosäuresequenz von SEQ ID NO:4 auf; oder
als Sequenz der variablen Region der schweren Kette weist die Aminosäuresequenz eine Homologie von 80 % oder mehr zur Aminosäuresequenz von SEQ ID NO:5 auf, und als Sequenz der variablen Region der leichten Kette weist die Aminosäuresequenz eine Homologie von 80 % oder mehr zur Aminosäuresequenz von SEQ ID NO:6 auf, und/oder
wobei der generische Antikörper umfasst:
als Sequenz der variablen Region der schweren Kette weist die Aminosäuresequenz eine Homologie von 80 % oder mehr zur Aminosäuresequenz von SEQ ID NO:7 auf, und als Sequenz der variablen Region der leichten Kette weist die Aminosäuresequenz eine Homologie von 80 % oder mehr zur Aminosäuresequenz von SEQ ID NO:8 auf; oder
als Sequenz der variablen Region der schweren Kette weist die Aminosäuresequenz eine Homologie von 80% oder mehr zur Aminosäuresequenz von SEQ ID NO:9 auf und als Sequenz der variablen Region der leichten Kette weist die Aminosäuresequenz eine Homologie von 80% oder mehr zur Aminosäuresequenz von SEQ ID NO:10 auf.

11. Verfahren zur Bestimmung des Grades der Kontamination durch Enterobacteriaceae-Bakterien in einer Probe, die aus Lebensmittel- oder Getränkeproben, Umweltproben und biologischen Proben ausgewählt ist, umfassend den Schritt des gleichzeitigen Nachweises des Vorhandenseins und/oder der Menge von Enterobacteriaceae-Bakterien aus zwei oder mehr Gattungen in der Probe auf der Grundlage von Antigen-Antikörper-Reaktionen durch das Verfahren nach einem der Ansprüche 1 bis 10.

12. Kit zum Nachweis des Vorhandenseins und/oder der Menge von Enterobacteriaceae-Bakterien in einer Probe, ausgewählt aus Lebensmittel- oder Getränkeproben, Umweltproben und biologischen Proben, durch das Verfahren nach einem der Ansprüche 1 bis 5, umfassend einen Antikörper, wie in einem der Ansprüche 1 bis 5 angegeben.

13. Kit zum Nachweis des Vorhandenseins und/oder der Menge von Enterobacteriaceae-Bakterien in einer Probe, die aus Lebensmittel- oder Getränkeproben, Umweltproben und biologischen Proben ausgewählt ist, durch das Verfahren nach einem der Ansprüche 6 bis 10, umfassend einen Einfang-Antikörper und einen Nachweis-Antikörper, wie in einem der Ansprüche 6 bis 10 angegeben, wobei entweder der Einfang-Antikörper oder der Nachweis-Antikörper der generische Antikörper und der andere der spezifische Antikörper ist,
gegebenenfalls umfasst der Kit ferner einen unlöslichen Membranträger zum Entwickeln der Probe und zum Inkontaktbringen der Probe mit dem Einfang-Antikörper, wobei der unlösliche Membranträger eine darauf gebildete Nachweislinie aufweist und der Einfang-Antikörper auf der Nachweislinie immobilisiert ist, wodurch zwei oder mehr Arten von Bakterien in der Probe auf der einzelnen Nachweislinie nachgewiesen werden.

14. Kit nach Anspruch 12 oder 13, bei dem es sich um einen Immunochromatographie-Kit handelt, wobei der Einfang-Antikörper und der Nachweis-Antikörper gegebenenfalls so ausgewählt sind, dass sie Antigen-Antikörper-Reaktionen mit dem Ribosomenprotein L7/L12 der in der Probe nachzuweisenden Enterobacteriaceae-Bakterien unter Bildung einer Sandwich-Struktur hervorrufen,
wobei der Immunochromatographie-Kit ferner umfasst:
einen unlöslichen Membranträger zum Entwickeln der Probe und Inkontaktbringen der Probe mit dem Einfang-Antikörper; und
ein Konjugatpolster, das auf dem unlöslichen Membranträger gebildet wird und an das der Nachweis-Antikörper gebunden ist, und
wobei der Einfang-Antikörper an dem Konjugat-Pad auf dem unlöslichen Membranträger entlang der Entwicklungsrichtung der Chromatographie immobilisiert ist.

## Revendications

1. Procédé de détection de la présence et/ou de la quantité de bactéries Enterobacteriaceae dans un échantillon choisi parmi les échantillons d'aliments ou de boissons, les échantillons environnementaux et les échantillons biologiques, le procédé comprenant l'étape de détection simultanée de la présence et/ou de la quantité de bactéries Enterobacteriaceae de deux genres différents ou plus dans l'échantillon sur la base de réactions antigène-anticorps,
dans lequel l'étape de détection comprend :
mise en contact de l'échantillon avec un anticorps qui provoque des réactions antigène-anticorps avec des composants dérivés des bactéries Enterobacteriaceae de deux genres ou plus ; et
mesurer la présence et/ou l'intensité de la réaction antigène-anticorps qui se produit dans l'échantillon après le contact,
dans lequel l'anticorps est un anticorps monoclonal ayant :
en tant que séquence de la région variable de la chaîne lourde, la séquence d'acides aminés ayant une homologie de 80 % ou plus avec la séquence d'acides aminés de SEQ ID NO:1, et en tant que séquence de la région variable de la chaîne légère, la séquence d'acides aminés ayant une homologie de 80 % ou plus avec la séquence d'acides aminés de SEQ ID NO:2 ; ou
en tant que séquence de la région variable de la chaîne lourde, la séquence d'acides aminés ayant une homologie de 80 % ou plus avec la séquence d'acides aminés de SEQ ID NO:3, et en tant que séquence de la région variable de la chaîne légère, la séquence d'acides aminés ayant une homologie de 80 % ou plus avec la séquence d'acides aminés de SEQ ID NO:4 ; ou
en tant que séquence de la région variable de la chaîne lourde, la séquence d'acides aminés ayant une homologie de 80 % ou plus avec la séquence d'acides aminés de SEQ ID NO:5, et en tant que séquence de la région variable de la chaîne légère, la séquence d'acides aminés ayant une homologie de 80 % ou plus avec la séquence d'acides aminés de SEQ ID NO:6.

2. Procédé selon la revendication 1, dans laquelle l'étape de détection comprend la détection simultanée de bactéries Enterobacteriaceae de deux ou plus, ou 3 ou plus, ou 4 ou plus, ou 5 ou plus, ou 6 ou plus, ou 7 ou plus genres différents choisis parmi le groupe constitué du genre Escherichia, du genre Klebsiella, du genre Citrobacter, du genre Enterobacter, du genre Proteus, du genre Salmonella, et du genre Serratia.

3. Procédé selon la revendication 1 ou 2, dans lequel les composants sont des protéines L7/L12 du ribosome des bactéries Enterobacteriaceae de deux genres ou plus.

4. Procédé selon la revendication 3, dans lequel l'anticorps ne provoque pas de réactions croisées avec des composants dérivés d'une ou de plusieurs bactéries autres que les entérobactéries qui peuvent être présentes dans l'échantillon,
éventuellement, dans lequel la ou les bactéries autres que les entérobactéries sont une ou plusieurs bactéries choisies parmi le genre Pseudomonas, le genre Staphylococcus, le genre Bacillus et le genre Enterococcus.

5. Procédé selon la revendication 3 ou 4, dans lequel l'anticorps ne provoque pas de réactions croisées avec au moins un composant non bactérien pouvant être présent dans l'échantillon,
éventuellement, dans lequel le composant non bactérien est un composant organique dérivé d'un virus, d'une plante et/ou d'un animal.

6. Procédé selon l'une des revendications 1 à 5, comprenant la détection de la présence et/ou de la quantité de bactéries Enterobacteriaceae dans l'échantillon par les étapes suivantes :
(I) capturer les bactéries dans l'échantillon et détecter les bactéries dans l'échantillon sur la base de réactions antigène-anticorps entre l'échantillon, un anticorps de capture lié à un support en phase solide, et un anticorps de détection ayant une étiquette de détection, et
(II) détection de la bactérie à détecter dans l'échantillon sur la base de l'étiquette de détection,
dans lequel l'un des anticorps de capture et l'anticorps de détection sont au moins un anticorps spécifique, qui provoque des réactions antigène-anticorps avec deux espèces de bactéries ou plus à détecter, et l'autre est au moins un anticorps générique, qui provoque des réactions antigène-anticorps avec des bactéries de cinq genres ou plus, y compris la bactérie à détecter, et
dans lequel l'anticorps générique ou l'anticorps spécifique est un anticorps tel que décrit dans l'une des revendications 1 à 5.

7. Procédé selon la revendication 6, dans lequel l'étape (I) comprend les étapes suivantes :
(Ia-1) mise en contact de l'anticorps de détection avec l'échantillon pour détecter les bactéries dans l'échantillon sur la base de la réaction antigène-anticorps entre l'anticorps de détection et les bactéries ; et
(Ia-2) mise en contact de l'anticorps de capture avec l'échantillon contenant les bactéries marquées par l'anticorps de détection afin de capturer les bactéries dans l'échantillon sur la base de la réaction antigène-anticorps entre l'anticorps de capture et le complexe bactérie-anticorps de détection, ou
dans laquelle l'étape (I) comprend les étapes suivantes :
(Ib-1) mise en contact de l'anticorps de capture avec l'échantillon pour capturer les bactéries dans l'échantillon sur la base de la réaction antigène-anticorps entre l'anticorps de capture et les bactéries, et
(Ib-2) mise en contact de l'anticorps de détection avec l'échantillon contenant les bactéries capturées par l'anticorps de capture afin de détecter les bactéries dans l'échantillon sur la base de la réaction antigène-anticorps entre l'anticorps de détection et le complexe bactérie-anticorps de capture.

8. Procédé selon la revendication 6 ou 7, dans laquelle l'anticorps de capture est l'anticorps générique et l'anticorps de détection est l'anticorps spécifique, ou
dans laquelle l'anticorps de détection est l'anticorps générique et l'anticorps de capture est l'anticorps spécifique.

9. Procédé selon l'une des revendications 6 à 8, dans lequel l'anticorps spécifique provoque des réactions antigène-anticorps avec des bactéries Enterobacteriaceae de deux genres ou plus choisis parmi au moins le genre Escherichia, le genre Klebsiella, le genre Citrobacter, le genre Enterobacter, le genre Proteus, le genre Salmonella, et le genre Serratia, et/ou
dans lequel l'anticorps générique provoque des réactions antigène-anticorps avec des bactéries d'au moins cinq genres choisis parmi au moins le genre Escherichia, le genre Klebsiella, le genre Citrobacter, le genre Enterobacter, le genre Proteus, le genre Salmonella, le genre Serratia, le genre Pseudomonas, le genre Staphylococcus, le genre Bacillus et le genre Enterococcus.

10. Procédé selon l'une des revendications 6 à 9, dans lequel l'anticorps spécifique comprend :
en tant que séquence de la région variable de la chaîne lourde, la séquence d'acides aminés présentant une homologie de 80 % ou plus avec la séquence d'acides aminés de SEQ ID NO:1, et en tant que séquence de la région variable de la chaîne légère, une séquence d'acides aminés présentant une homologie de 80 % ou plus avec la séquence d'acides aminés de SEQID NO:2; ou
en tant que séquence de la région variable de la chaîne lourde, la séquence d'acides aminés présentant une homologie de 80 % ou plus avec la séquence d'acides aminés de SEQ ID NO:3, et en tant que séquence de la région variable de la chaîne légère, une séquence d'acides aminés présentant une homologie de 80 % ou plus avec la séquence d'acides aminés de SEQID NO:4; ou
en tant que séquence de la région variable de la chaîne lourde, la séquence d'acides aminés ayant une homologie de 80 % ou plus avec la séquence d'acides aminés de SEQ ID NO:5, et en tant que séquence de la région variable de la chaîne légère, une séquence d'acides aminés ayant une homologie de 80 % ou plus avec la séquence d'acides aminés de SEQ ID NO:6, et/ou
dans lequel l'anticorps générique comprend
en tant que séquence de la région variable de la chaîne lourde, la séquence d'acides aminés ayant une homologie de 80 % ou plus avec la séquence d'acides aminés de SEQ ID NO:7, et en tant que séquence de la région variable de la chaîne légère, une séquence d'acides aminés ayant une homologie de 80 % ou plus avec la séquence d'acides aminés de SEQ ID NO:8 ; ou
en tant que séquence de la région variable de la chaîne lourde, la séquence d'acides aminés ayant une homologie de 80 % ou plus avec la séquence d'acides aminés de SEQ ID NO:9, et en tant que séquence de la région variable de la chaîne légère, une séquence d'acides aminés ayant une homologie de 80 % ou plus avec la séquence d'acides aminés de SEQ ID NO:10.

11. Procédé pour déterminer le degré de contamination par les bactéries Enterobacteriaceae dans un échantillon choisi parmi les échantillons d'aliments ou de boissons, les échantillons environnementaux et les échantillons biologiques, comprenant l'étape de détection simultanée de la présence et/ou de la quantité de bactéries Enterobacteriaceae de deux genres ou plus dans l'échantillon sur la base de réactions antigène-anticorps par la Procédé selon l'une quelconque des revendications 1 à 10.

12. Kit pour détecter la présence et/ou la quantité de bactéries Enterobacteriaceae dans un échantillon choisi parmi les échantillons d'aliments ou de boissons, les échantillons environnementaux et les échantillons biologiques par le procédé selon l'une des revendications 1 à 5, comprenant un anticorps tel que décrit dans l'une des revendications 1 à 5.

13. Kit pour détecter la présence et/ou la quantité de bactéries Enterobacteriaceae dans un échantillon choisi parmi les échantillons d'aliments ou de boissons, les échantillons environnementaux et les échantillons biologiques par le procédé selon l'une des revendications 6 à 10, comprenant un anticorps de capture et un anticorps de détection comme indiqué dans l'une des revendications 6 à 10, dans lequel l'un de l'anticorps de capture et de l'anticorps de détection est l'anticorps générique et l'autre est l'anticorps spécifique,
éventuellement, le kit comprend en outre un support membranaire insoluble pour développer l'échantillon et mettre en contact l'échantillon avec l'anticorps de capture, dans lequel le support membranaire insoluble comporte une ligne de détection et l'anticorps de capture est immobilisé sur la ligne de détection, ce qui permet de détecter deux espèces de bactéries ou plus dans l'échantillon sur la seule ligne de détection.

14. Kit selon la revendication 12 ou 13, qui est un kit d'immunochromatographie, éventuellement dans lequel l'anticorps de capture et l'anticorps de détection sont sélectionnés de manière à provoquer des réactions antigène-anticorps avec la protéine ribosomique L7/L12 de la bactérie Enterobacteriaceae à détecter dans l'échantillon pour former une structure en sandwich,
dans lequel le kit d'immunochromatographie comprend en outre :
un support membranaire insoluble pour développer l'échantillon et mettre en contact l'échantillon avec l'anticorps de capture ; et
un tampon conjugué formé sur le support membranaire insoluble et auquel l'anticorps de détection est attaché, et
dans lequel l'anticorps de capture est immobilisé sur le tampon conjugué sur le support membranaire insoluble le long de la direction de développement de la chromatographie.
